(19) **Europäisches Patentamt**
**European Patent Office**
**Office européen des brevets**

(11) **EP 4 207 204 B1**

(12) **EUROPEAN PATENT SPECIFICATION**

(45) Date of publication and mention
of the grant of the patent:
**23.07.2025   Bulletin 2025/30**

(21) Application number: **22217441.9**

(22) Date of filing: **31.12.2022**

(51) International Patent Classification (IPC):
**G16B 20/20** *(2019.01)*       **G16B 40/30** *(2019.01)*

(52) Cooperative Patent Classification (CPC):
**G16B 20/20; G16B 40/30**

(54) **METHODS AND SYSTEMS FOR DETECTING TUMOR MUTATIONAL BURDEN**

VERFAHREN UND SYSTEME ZUR ERKENNUNG EINER TUMORMUTATIONSLAST

PROCÉDÉS ET SYSTÈMES DE DÉTECTION DE CHARGE MUTATIONNELLE TUMORALE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **31.12.2021   US 202163295709 P
30.12.2022   US 202218091866**

(43) Date of publication of application:
**05.07.2023   Bulletin 2023/27**

(73) Proprietor: **Sophia Genetics S.A.
1180 Rolle (CH)**

(72) Inventors:
• **Bieler, Jonathan
1025 Saint-Sulpice (CH)**
• **Wenger, Yvan
1025 Saint-Sulpice (CH)**
• **Pozzorinni, Christian
1025 Saint-Sulpice (CH)**
• **Xu, Zhenyu
1025 Saint-Sulpice (CH)**

(74) Representative: **IK-IP LTD
3 Lloyd's Avenue
London, Greater London EC3N 3DS (GB)**

(56) References cited:
**WO-A1-2020/092591       WO-A2-2021/247902
US-A1- 2019 362 808       US-A1- 2020 202 224**

• **CHOO-WOSOBA HYOYOUNG ET AL: "A Hidden
Markov Modeling Approach for Identifying
Tumor Subclones in Next-Generation
Sequencing Studies", BIORXIV, 20 June 2019
(2019-06-20), XP093047349, Retrieved from the
Internet <URL:https://www.biorxiv.org/content/
10.1101/675512v1.full.pdf> [retrieved on
20230516], DOI: 10.1101/675512**
• **GENG YU ET AL: "An Expanded Association
Approach for Rare Germline Variants with Copy-
Number Alternation", 1 April 2017, SAT 2015
18TH INTERNATIONAL CONFERENCE, AUSTIN,
TX, USA, SEPTEMBER 24-27, 2015; [LECTURE
NOTES IN COMPUTER SCIENCE; LECT.NOTES
COMPUTER], SPRINGER, BERLIN,
HEIDELBERG, PAGE(S) 81 - 94, ISBN:
978-3-540-74549-5, XP047454537**

**Description**

FIELD OF THE INVENTION

Broadly, methods and systems described herein relate to genomic analysis, however, more specifically, methods and systems described herein relate to the use of genomic information for detecting and cancer. While the method is not itself a method for treatment, the data produced may be useful in guiding treatment.

BACKGROUND

Tumor Mutational Burden As A Predictor Of Anticancer Treatment Responses

Tumorigenesis can be associated with a mutator phenotype, where somatic mutations accumulate at unusually high rates. Biological samples from cancerous patients may be analyzed with a high throughput sequencing workflow to characterize the number of somatic mutations per a particular length (i.e., a megabase) of an interrogated genomic sequence, which can be used as an indication of the tumor mutational load or tumor mutational burden (TMB). TMB is a predictive biomarker for the response to a diversity of immunotherapy treatments for various cancers, such as melanoma and lung cancer, and respective patients' response to immune checkpoint inhibitors (ICI) (Snyder et al, N England J Med, 2014 Dec 4; 371 (23); Rivzi et al, Science, 2015 Apr 3; 348(6230)).

TMB Genomic Analysis Assays

Accordingly, a method of estimating patient tumor mutational burden from DNA samples, obtained, for example, from a tumor, may therefore facilitate the choice of the most suitable cancer treatment to patients in accordance with the characterization of the number of somatic mutations identified in DNA obtained from their tumor samples or cell free DNA. The genomic sequencing workflow may employ Whole Genome Sequencing (WGS), Whole Exome Sequencing (WES), or targeted panels (Campesato et al, Oncotarget, vol. 6, no. 33, 2015) in combination with a bioinformatics workflow suitable for the analysis of the genomic sequencing data. Identification of whether a sample carries a high number of somatic mutations (TMB-high) may be of considerable assistance in the planning of treatment. Multiple research and commercial providers have developed TMB estimation assays based on targeted assays over the past five years. The United States Food and Drug Association has, for instance, approved the use of the Foundation Medicine CDx assay to determine a TMB-high status, measured as the count of at least 10 somatic mutations per megabase (10 mut/Mb) as a predictor of pembrolizumab for the treatment of some adult and pediatric cancer patients. In "Aligning Tumor Mutational Burden (TMB) quantification across diagnostic samples: Phase 2 of the Friends of Cancer Research TMB harmonization project", Annals of Oncology (2021), published online Sep 30, 2021, a consortium of international experts ("The TMB Harmonization Project") reviewed the TMB targeted panel assays from 16 laboratories and compared their results against those of WES on a selection of 29 tumor samples and 10 human-derived cell lines representative of different cancers (e.g., bladder, colon, gastric, gastrointestinal stromal tumor (GIST), lung, and breast). Of the aforementioned 16 laboratories, most (14) used approaches that rely solely on the analysis of DNA samples from a patient tumor, hereafter referred to as the tumor-only approach, to identify the somatic mutations. Tumor-only approaches bypass the need of the patient germline reference sample to filter out the germline variants prior to TMB estimation and are advantageous in routine clinical practice because they do not require the need to reconcile the NGS analysis of the tumor sample DNA, which contains both germline and somatic variants and is often extracted from Formalin-Fixed Paraffin-Embedded (FFPE) tumor samples, with the NGS analysis of a separate germline sample from the same patient, usually, extracted from peripheral blood cells. However, and as a consequence of the absence of a non-tumor sample from the same patient, the somatic-only assays require a dedicated data processing strategy to identify and remove the germline variants from the tumor sample DNA NGS analysis results. This may be performed in the bioinformatics genomic analysis workflow by using databases of common germline variants, such as the single Nucleotide Polymorphism Database (dbSNP), Exome aggregation consortium (ExAC), the 1000 Genome Project Database, the Genome Aggregation Consortium (GnomAD), the ESP5400 data of the National Heart, Lung and Blood Institute GO Exome Sequencing Project, the Cancer Genome Atlas (TCGA) and other suitable databases. However, such databases are not comprehensive and are often biased for certain populations, leading to certain germline variants in the samples being considered as somatic (Kessler et al, Nature Comms, 2016 Oct. 11; 12521(7)). This misassignment ultimately results in overestimation of the TMB count (Garofalo et al., "The impact of tumor profiling approaches and genomic data strategies for cancer precision medicine", Genome Medicine (2016) 8:79). In addition, the fraction of the genome that is captured by the targeted-capture panel also impacts the TMB measurement, particularly when paired germline sequencing data is not available. In particular, TMB analysis from tumor-only data is most accurate when large targeted-capture panels are used in combination with comprehensive and ethnically diverse germline variant databases. (Garofalo et al., "The impact of tumor profiling approaches and genomic data strategies for cancer precision medicine", Genome Medicine (2016) 8:79). In sum, sizable tumor-only targeted panels may be adequate for the majority of somatic variant identification and mutational load prediction if paired with expanded germline analysis approaches. Paired germline sequencing may reduce overall false positive mutation calls and WES may provide the most neoantigens. In general, without patient-matched germline data, large germline databases may be desired to lessen false positive mutation calling and alleviate categorical disparities.

WO2018106884 by Life Technologies describes methods to filter out somatic variants identified in DNA samples from the patient present in one or more population genetic variant databases, by only retaining the variants with a minor allele frequency (MAF) within a predetermined MAF range, typically a low range. This enables the workflow to retain the rarest variants as somatic variants while excluding the more common germline variants present in the reference database records. While the described methods of WO2018106884 enable removal of some false positive somatic mutations as the most commonly found ones, this filter still relies upon the relevance of the reference databases for variant annotation without taking into account the individual genomic patterns and sample properties into its heuristics. Many databased may be inherently limiting because many such databases contain variants that have been found in the germline of profiled individuals. Accordingly, as a large fraction of this germline variant may be common, this may provide a useful source of information. However, the methods described herein may utilize information from such databases and/or may optimize or otherwise supplement such information. Yet, many germline databases contain a number of, but certainly not all, germline variants. For example, this may be particularly true for variants found in certain ethnic groups that are less represented in a particular study. Further, traditional methods may assume that somatic variants are found at low frequency; while this may often be true, a number of factors, such as genomic patterns and sample properties, can impact this.

WO2020092591 by Illumina describes methods to discriminate a somatic variant from a germline variant that take into account the allele frequency of the variant in the DNA sample and its location in the genome. This method relies on the steps of binning variants located in the same region of the genome; identifying a first set of germline variants in the bin that are also present in population genetics databases; and applying a proximity filter to identify a second set of germline variants in the same bin as those with an allele frequency within a range of the first germline variants in the bin . While this method provides a finer spatial granularity on certain genomic regions, it still fails to consider the individual genomic patterns, as well as certain inter-region dependencies, such as the likelihood of linkage disequilibrium between a number of human genome mutations. Moreover, predefined bins can be problematic if the sample contains more or fewer copy number variations (CNVs) than expected (i.e., variable ploidy overlapping the predefined bins) or multiple tumor clones. Specifically, such a traditional method may assume that the dependency of the variant fraction is constant and can be captured by a bin of a certain size. While in some instances this may be a decent approximation, such a traditional method fails to capture situations where the dependencies are broken by common genomic events, such as linkage disequilibrium and the presence of CNVs.

WO2017151524 by Foundation Medicine also proposes a filtering method to evaluate the TMB count by using a cascade of filtering steps. First, the germline mutations found in the DNA from the sample are filtered out by using the somatic-germline-zygosity (SGZ) algorithm (Sun et al, PLoS Comput Biol 14(2) (2018)) with reference to a population genetic variant reference database. The SGZ algorithm predicts the somatic versus germline status of each alteration identified by modeling the variant allele frequency (VAF), taking into account the tumor content (sample purity), the tumor ploidy, and the local copy number. Second, this SGZ algorithm can be extended in the bioinformatics genomic analysis workflow by further using databases of somatic variants with a known functional status, such as the COSMIC database. By using this additional information, the method further filters out the functional mutations that may influence cell division, growth, or survival. This method preferably operates in combination with a targeted panel rather than with a WGS or a WES assay. Moreover, out of the targeted panel sequencing data, some specific genomic regions may also be excluded from the TMB count, such as regions corresponding to certain genes, e.g., tumor genes. One major drawback of this method is that it requires prior knowledge on sample ploidy and purity that is not commonly available and is difficult to infer. Accordingly, such traditional methods may be limited as they may require prior knowledge of sample ploidy and purity.

US 2019/362808 A1 relates to methods for detecting somatic and germline variants in tumour samples using sequencing data, particularly in cases where normal tissue samples are unavailable. D1 discloses an initial step of filtering out known germline variants based on population databases and further analysing so-called "private germline variants" not found in databases that would be incorrectly classified as somatic variants in tumour-only sequencing. To this end, Bayesian modelling is used, as well as expectation maximization, and machine-learning algorithms to differentiate between somatic and germline variants, considering factors like copy number alterations and tumour cell fraction. US 2019/362808 A1 does not disclose the use of a Hidden Markov Model along each chromosome to determine the likelihood that a candidate somatic variant is a germline variant.

There is therefore a need for improved tumor mutational burden estimation methods that are suitable for use in a genomic analysis workflow for a next generation sequencing experiment, and which can better discriminate somatic mutations from germline mutations for a diversity of individual genomic patterns without the need for a matched tumor-normal, even when the germline reference databases are not fully representative of the individual patient germline genomics.

SUMMARY OF THE INVENTION

The invention is set out in the appended claims. This Summary is provided to introduce a selection of concepts in a simplified form that are further described below in the Detailed Description. This Summary is not intended to identify key features or essential features, nor is it intended to limit the scope of the claims included herewith.

The invention of the present disclosure may be a computer-implemented method of determining a tumor mutational burden (TMB) value in a tumor sample, the method comprising obtaining variant calling data for the tumor sample; and identifying, in the variant calling data and in view of at least one population database, a list of germline variants for the tumor sample along each chromosome, wherein each of the germline variants of the list of germline variants includes a population minor allele frequency (pMAF) above a threshold relative to the at least one population database. In an embodiment, the computer-implemented method may further comprise identifying, in the variant calling data, a list of candidate somatic variants, wherein the list of candidate somatic variants are the remaining variants in the variant calling data that have not been identified in the list of germline variants. The computer-implemented method may further comprise filtering out likely germline variants from the list of candidate somatic variants to retain only likely somatic variants. In such an embodiment, filtering out the likely germline variants may further comprise the steps of estimating, via a Hidden Markov Model (HMM) along each chromosome, a probability of each candidate somatic variant *i* being a germline variant ("Pgermline(*i*)"); and determining, based on the Pgermline(*i*), whether a candidate somatic variant *i* is germline or somatic, to retain only the likely somatic variants in the list of candidate somatic variants. In another embodiment, the computer-implemented method comprises determining the tumor mutational burden (TMB) value for the tumor sample based on a count of the retained likely somatic variants over all chromosomes.

In an embodiment, observed states for the HMM along each chromosome comprise variant fractions from the list of germline variants, and the variant fractions from the list of germline variants are obtained from sequencing data. In another embodiment, hidden states for the HMM along each chromosome comprise at least 9 discretized variant fractions. The HMM model may employ a transition probability ($p_{switch}$) representing the probability that the two subsequent variants, *i-1* and *i,* along a chromosome, with variant fractions, $VF_{i-1}$ and $VF_i$, respectively, are associated to the same hidden state. As a non-limiting example, $p_{switch}$ is chosen in a range from 1e-3 to 1e-6. As another non-limiting example, $p_{switch}$ is a value of 2e-4.

In an embodiment, emission probabilities of the HMM are defined as:

$$P\big(VF_O(i)\big|VF_H(i)\big) = 0.9 \times \text{Beta}\big((\alpha, \beta); VF_O(i)\big) + 0.1 \times \text{Uniform}\big(0, 1; VF_O(i)\big)$$

wherein $\alpha = 1 + VF_H(i)$; wherein $\beta = 1 + D * (1 - VF_H(i))$; wherein $D$ is a mean coverage depth of variants in the tumor sample; and $VF_O(i)$ is an observed variant fraction originating from hidden state $VF_H(i)$.

In one embodiment, the mean coverage depth $D$ is rescaled by multiplying the mean coverage depth $D$ by a factor chosen in a range of 1/6 to 1/2. In an alternate embodiment, the mean coverage depth $D$ is rescaled by multiplying the mean coverage depth $D$ by a factor of 1/4. In yet another embodiment, the mean coverage depth D is rescaled by multiplying the mean coverage depth D by a factor chosen in a range of 1/2 to 1.

In an embodiment, for each chromosome, the HMM receives as input the list of heterozygous germline variants along said chromosome by retaining only those heterozygous germline variants observed with an observed variant fraction $VF_O(i)$ within a predetermined range of above 5% and below 90%. Further, if the observed variant fraction $VF_O(i)$ of the heterozygous germline variants is equal to or greater than 50%, then the observed variant fraction $VF_O(i)$ may be replaced by observed state value 1-VF.

The computer-implemented method may further include adding one or more artificial data points at one or more chromosome genomic coordinates of regions with no observed variant calling data prior to estimating via the HMM. In such an embodiment, a regular grid is used in adding the one or more artificial data points, and the regular grid may comprise a resolution configured to maintain a predetermined number of grid points between a majority of observed variants.

In an embodiment, filtering out the likely germline variants from the list of candidate somatic variants comprises retaining only the candidate somatic variants for which the Pgermline(*i*) is below a threshold value (Pgermline-ThreshHMM). The PgermlineThreshHMM may be 1e-3. Alternatively, the PgermlineThreshHMM may be 1e-4.

The computer-implemented method may further comprise reporting to an end user the TMB value for the tumor sample.

In an embodiment, obtaining the variant calling data for the tumor sample comprises sequencing the tumor sample. In another embodiment, a TMB value may be determined in the cancer patient tumor sample according to the computer-implemented method described herein.

In an alternate embodiment, a computer-implemented method is proposed for determining a tumor mutational burden (TMB) value in a tumor sample, the method comprising obtaining variant calling data from Next Generation Sequencing data for the tumor DNA sample; identifying (for the purposes of this disclosure, "identifying" may refer to searching for the presence or absence of a variant found in the tumor DNA sample [sequence and genomic location] in view of the information [genomic location and sequence] found in the population genetics/germline variant database), in the variant calling data and at least one population database, a list of germline variants for the tumor DNA sample along each chromosome; identifying, in the variant calling data, a list of candidate somatic variants as the variants in the variant calling data that have not been identified in the list of germline variants; filtering out likely germline variants from the list of candidate somatic variants to retain only somatic variants; determining the tumor mutational burden (TMB) value for the tumor sample from the count of the retained somatic variants over all chromosomes. In a further embodiment, the method may comprise the step of identifying amongst the likely somatic variants and at least one database containing cancer driving mutations, a list of cancer driving mutations and further filtering said cancer driving mutations. In one embodiment, filtering out of germline variants from the list of candidate somatic variants to retain only somatic variants comprises estimating, with a Hidden Markov Model (HMM) along each chromosome, the probability of each candidate somatic variant $i$ being a germline variant (Pgermline($i$)); and determining, from the probability Pgermline($i$), whether a candidate somatic variant $i$ is germline or somatic, to retain only the likely somatic variants in the list of candidate somatic variants.

In one embodiment, the observed states for the HMM along each chromosome comprise the variant fractions obtained from the variant calling data for the list of germline variants.

In one embodiment, the observed states for the HMM along each chromosome comprise the variant fractions (VF) of heterozygous germline variants, the VFs selected in a range from 5% to 90%. In a further embodiment, if the VF is above 50%, it may be replaced by observed state value 1-VF. In another embodiment, germline variants may be compared to a database and may be utilized to define the aforementioned range.

In one embodiment, artificial data points may be added at chromosome genomic coordinates of regions with no observed variant calling data prior to applying the HMM. A regular grid may be used when adding artificial data points, wherein the resolution of the grid is chosen such that there are several grid points between the majority of observed variants.

In one embodiment, filtering out likely germline variants from the list of candidate somatic variants comprises retaining only the candidate somatic variants for which the probability Pgermline($i$) is below a threshold value Pgermline-ThreshHMM.

In one embodiment, the estimated tumor mutational burden value is reported to an end user.

In one embodiment, a method for treatment selection for a cancer patient comprises a step of determining a TMB value in the cancer patient tumor sample according to some embodiments of the proposed computer-implemented methods.


BRIEF DESCRIPTION OF THE DRAWINGS

Objects, aspects, features, and advantages of embodiments disclosed herein will become more fully apparent from the following detailed description, the appended claims, and the accompanying drawing figures in which like reference numerals identify similar or identical elements. Reference numerals that are introduced in the specification in association with a drawing figure may be repeated in one or more subsequent figures without additional description in the specification in order to provide context for other features, and not every element may be labeled in every figure. The drawing figures are not necessarily to scale, emphasis instead being placed upon illustrating embodiments, principles and concepts. The drawings are not intended to limit the scope of the claims included herewith.

FIG. 1 represents a next generation sequencing system according to certain embodiments of the present disclosure.
FIG. 2 represents a genomic analysis workflow for estimating and reporting a tumor mutational burden value from the variant calling data of a patient DNA sample without the need for a germline reference.
FIG. 3 represents an improved somatic variants identification workflow according to some embodiments of the present disclosure.
FIGS. 4A-B shows examples of a) pre-classified germline versus candidate somatic variants and b) filtered germline versus somatic variants with an embodiment of the Hidden Markov Model along different chromosomes.

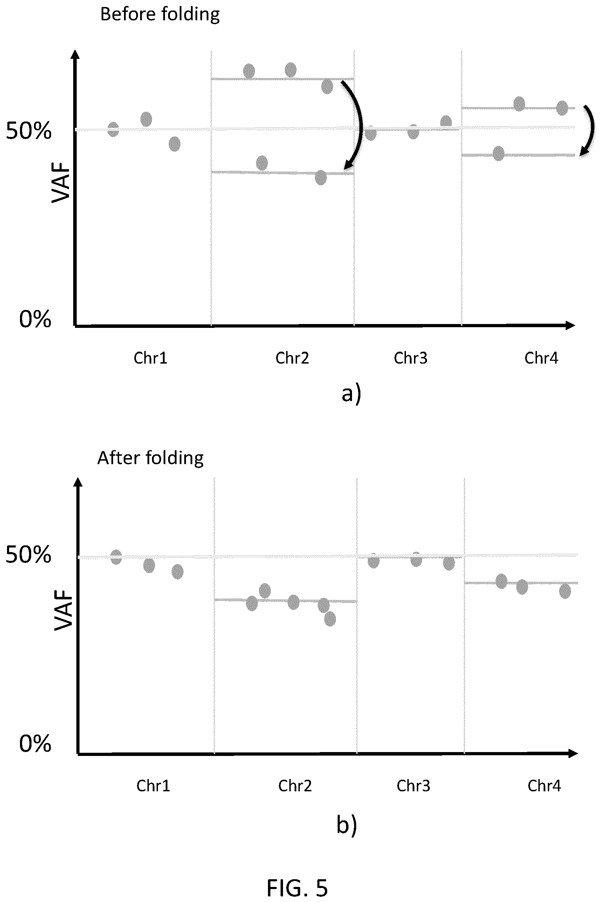

FIG. 5 schematically illustrates a VF folding pre-processing of the input data to further optimize the an embodiment of the Hidden Markov Model efficiency.
FIG. 6A shows the resulting classification of variants as germline or somatic according to an experiment with some of the proposed embodiments.
FIG. 6B shows the relationship between TMB results obtained using an embodiment of the method of the proposed implementation (implementation one) and a method used as reference (implementation two).
FIG. 7 shows the germline variants used as input for the HMM model (grey points), and for the remaining candidate somatic variants, in black, the output of the HMM filter classified as either somatic variants (black triangles) or germline variants (black points). The line contours represent the posterior distribution of the inferred VF of the germlines, which may be separately calculated by the HMM for each chromosome.

FIG. 8 shows a simulation of the impact of copy number loss and gain in view of the loss of an allele (CN1); a copy neutral loss of heterozygosity (CN2*); and an allele duplication (CN3)

FIG. 9 shows the resulting distribution of the VFs of simulated variants following an implementation of an embodiment of the method along genomic coordinates for germline and somatic variants.

FIG. 10 shows the consideration of the number of somatic variants identified present on coding sequences and the total coding sequence length of 3.32 megabases covered by simulations to estimate TMB.

FIG. 11 shows consideration of a variant as germline if Pgermline exceeded a threshold of 1e-3.

FIG. 12 is an illustrative block diagram of a system based on a computer for execution of the methods described herein.

FIG. 13 is an illustration of a computing machine for execution of the methods described herein.

## DETAILED DESCRIPTION

In the following detailed description, reference will be made to the accompanying drawing(s), in which identical functional elements are designated with like numerals. The aforementioned accompanying drawings show by way of illustration, and not by way of limitation, specific aspects, and implementations consistent with principles of this disclosure. These implementations are described in sufficient detail to enable those skilled in the art to practice the disclosure and it is to be understood that other implementations may be utilized and that structural changes and/or substitutions of various elements may be made without departing from the scope of this disclosure. The following detailed description is, therefore, not to be construed in a limited sense.

It is noted that description herein is not intended as an extensive overview, and as such, concepts may be simplified in the interests of clarity and brevity.

Any process described in this application may be performed in any order. Processes may also be combined with other processes or steps of other processes.

The present disclosure is based, at least in part, on the concept that the presently disclosed genomic analyzer methods and systems, designed, in part, to process the variant calling data obtained from a tumor sample DNA, can discriminate between somatic and germline variants without the need for a germline sample reference to estimate a tumor mutational burden biomarker value. In particular, the proposed methods and systems are robust enough to be deployed in a data-driven medicine platform regardless of the diversity of tumor sample purities and ploidies as met in routine clinical practice.

The presently disclosed genomic analyzer methods and systems may be designed, in part, to process the variant calling data obtained from a tumor sample DNA, and may discriminate between somatic and germline variants without the need for a germline sample reference. Yet, somatic variants may be used to estimate a tumor mutational burden biomarker value.

The proposed methods and systems will now be described by reference to more detailed embodiments. The proposed methods and systems may, however, be embodied in different forms and should not be construed as limited to the embodiments set forth herein. Rather, these embodiments are provided so that this disclosure will be thorough and complete, and will fully convey the scope to those skilled in the art.

*Definitions*

A **"DNA sample"** refers to a nucleic acid sample derived from an organism, as may be extracted for instance from a solid tumor or a fluid. The organism may be a human, an animal, a plant, fungi, or a microorganism. The nucleic acids may be found in a solid sample such as a Formalin-Fixed Paraffin-Embedded (FFPE) sample. Further, nucleic acids may be found in a fresh-frozen sample. Alternately, the nucleic acids may be found in limited quantity or low concentration, in bodily fluids (e.g., blood or plasma). As circulating free DNA in bodily fluids. Circulating free DNA can include DNA shed from a tumor in a cancer patient (circulating tumor DNA).

A **"nucleotide sequence"** or a **"polynucleotide sequence"** refers to the sequence of nucleotides, such as cytosine (represented by the C letter in the sequence string), thymine (represented by the T letter in the sequence string), adenine (represented by the A letter in the sequence string), guanine (represented by the G letter in the sequence string) and uracil (represented by the U letter in the sequence string) in any polymer or oligomer of nucleotides. It may be DNA or RNA, or a combination thereof. It may be found permanently or temporarily in a single-stranded or a double-stranded shape. Unless otherwise indicated, nucleic acids sequences are written left to right in 5' to 3' orientation.

**"Sequencing"** refers to the process of determining the sequence of a polymer or oligomer of nucleotides.

**"High Throughput Sequencing (HTS)"** or **"Next Generation Sequencing (NGS)"** refers to real time sequencing of multiple sequences in parallel, typically between 50 and a few thousand base pairs per sequence. Exemplary NGS technologies include those from Illumina, Ion Torrent Systems, Oxford Nanopore Technologies, Complete Genomics, Pacific Biosciences, BGI, and others. Depending on the actual technology, NGS sequencing may require sample preparation with sequencing adaptors or primers to facilitate further sequencing steps, as well as amplification steps so that multiple instances of a single parent molecule are sequenced, for instance with PCR amplification prior to delivery to a flow cell in the case of sequencing by synthesis. HTS and NGS of a DNA library will produce a set of sequencing read data which can be processed by a bioinformatics computer in a bioinformatics workflow.

**"Sequencing depth"** or **"sequencing coverage"** or **"depth of sequencing"** refers to the number of times a genome has been sequenced. In targeted panels assay workflows, only a small subset of regions of interest in the whole genome is sequenced and it may therefore be reasonable to increase the sequencing depth without facing significant data storage and data processing overhead. Moreover, apart from the higher cost related to data storage and processing, the operational cost of an experimental NGS run, that is, loading a sequencer with samples for sequencing, also needs to be optimized by balancing the coverage depth and the number of samples which may be assayed in parallel in routine clinical workflows. Indeed, next generation sequencers are still limited in the total number of reads that they can produce in a single experiment (i.e., in a given run). The lower the coverage, the fewer reads per sample for the genomic analysis, and the higher the number of samples that can be multiplexed within a next generation sequencing run.

**"Aligning"** or **"alignment"** or **"aligner"** refers to mapping and aligning base-by-base, in a bioinformatics workflow, the sequencing reads to a reference genome sequence, depending on the application. For instance, in a targeted enrichment application where the sequencing reads are expected to map to a specific targeted genomic region in accordance with the hybrid capture probes used in the experimental amplification process, the alignment may be specifically searched relative to the corresponding sequence, defined by genomic coordinates such as the chromosome number, the start position and the end position in a reference genome. As known in bioinformatics practice, in some embodiments "alignment" methods as employed herein may also comprise certain pre-processing steps to facilitate the mapping of the sequencing reads and/or to remove irrelevant data from the reads, for instance by removing non-paired reads, and/or by trimming the adapter sequence as the end of the reads, and/or other read pre-processing filtering means. Exemplary bioinformatics data representations with different coordinate systems (absolute or relative position indexing, 0-based or 1-based, etc.) include the BED format, the GTF format, the GFF format, the SAM format, the BAM format, the VCF format, the BCF format, the Wiggle format, the GenomicRanges format, the BLAST format, the GenBank/EMBL Feature Table format, and other suitable formats.

**"Coverage"** or **"sequence read coverage"** or **"read coverage"** refers to the number of sequencing reads that have been aligned to a genomic position or to a set of genomic positions. In general, a genomic region with a higher coverage is associated with a higher reliability in downstream genomic characterization, in particular when calling variants.

A **"Bin"**, a **"genomic section"**, a **"partition"**, a **"genomic portion"**, or a **"portion of a chromosome"** refers to a contiguous region of interest of a genome. As such a region may include variants, a bin generally refers to the location or region of the genome rather than to a fixed DNA sequence. In bioinformatics methods and processes, a bin may be identified by its start and end genomic coordinates along the reference genome, and the bin length may be measured as a number of bases (b, kb, Mb) or basepairs (bp, kbp, Mbp) from the start to the end coordinates. In general, a bin may correspond to an entire chromosome, a segment of a chromosome, a segment of a reference genome, multiple chromosome portions, multiple chromosomes, portions from multiple chromosomes, and/or combinations thereof. Preferably, a bin is a portion of a chromosome obtained from partitioning of a reference genome into genomic sections (e.g., partitioned by size, segments, contiguous regions, contiguous regions of an arbitrarily defined size, and the like). Genomic sections may be selected, sorted, filtered and/or removed from consideration using any suitable criteria know in the bioinformatics. Along a genome, the bins can have the same, uniform length or different, variable lengths.

**"Variant calling"** refers to the process of identifying, in a bioinformatics workflow, the sequence variants in the aligned reads relative to a reference sequence. In bioinformatics data processing, a variant is uniquely identified by its position along a chromosome (*chr,pos*) and its difference relative to a reference genome at this position (*ref, alt*). Variants may include single nucleotide permutations (SNPs) or other single nucleotide variants (SNVs), insertions or deletions (INDELs), copy number variants (CNVs), as well as large rearrangements, substitutions, duplications, translocations, and others. Preferably variant calling is robust enough to sort out the real sequence variants from variants introduced by the amplification and sequencing noise artifacts, for example. In a bioinformatics workflow, a variant caller may apply variant calling to produce one or more variant calls listed in any suitable format, such as Variant Calling File (VCF format). However, other file formats may be utilized. It is understood that 'variant calling data' is the result of variant calling analysis according to any known method of variant calling. In the described methods, variant calling data may be data obtained from sequencing of a cancer patient tumor sample. As non-limiting examples, the present method may use variant calling data obtained with a next generation sequencing targeted panel, whole exome sequencing assay, or whole genome sequencing assay.

**"Mutation"** refers to a type of alteration in a nucleic acid sequence, such as a SNP, SNV or indel. A **"variant"** is a specific mutation occurring at a specific genomic position. A germline **variant** is a variant inherited from at least one individual parent that differs from the wildtype genomic value as registered in a reference database, and that is present in all somatic cells of the individual. A **somatic variant** or a **somatic mutation** or a **somatic alteration** is a variant caused by a genomic alteration, that is present in one or more somatic cells of the individual, for example in tumor cells.

**"Variant allele fraction"** or **"Variant allele frequency"** or **"VAF"** or "Variant fraction" or "VF" is a measure of the fraction of DNA molecules in an original specimen carrying a variant. VF can be measured in an NGS experiment by counting the number of sequencing reads that support a genomic variant divided by the overall coverage at that genomic position. Depending on the NGS assay, more sophisticated approaches may be used to measure VF. For instance, when unique molecular identifiers (UMIs) are used, polymerase chain reaction (PCR) duplicates may be identified, and the VF may be measured by counting the fraction of unique molecules supporting the variant, rather than the fraction of sequencing reads. Variant fraction estimated by NGS based on the number of sequencing reads supporting the presence of a given variant may deviate from the frequency at which a variant is present in the DNA sample.

A **"mutational load"** or **"mutation load"** or **"mutation burden"** or **"mutational burden",** or for a tumor a **"tumor mutational burden"** or **"tumor mutational load"** or **"TMB"** is measured as the number of somatic mutations per megabase of an interrogated genomic sequence.

A **"Markov model"** is a statistical model used to model randomly changing systems. A **"Hidden Markov Model"** or **"HMM"** is a Markov model with unobserved (i.e., hidden) states. A hidden Markov model can be represented as the simplest dynamic Bayesian network. In an embodiment, an HMM model enables estimation of a **hidden state** from an **observation state,** based on 1) pre-defined **emission probabilities** from the hidden states to the observation states; and 2) pre-defined **transition probabilities** between the hidden states themselves.

A **"population database"** refers to any database with genome sequencing data. Examples include, for instance, GnomAD, 1000genome (g1000), Exome Variant Server, dbSNP, dbVar, Personalized Genome Project, ExAC, esp5400, or other suitable databases. Use of a population database allows identification of a list of germline variants, wherein the list comprises at least 1, at least 100, or at least 1000 variants, organized along each chromosome.

A **"candidate somatic variant"** or a **"possible somatic variant"** or a **"possibly somatic variant"** refers to any variant that is found in the variant calling data but not in the list of identified germline variants for a sample.

Genomic Analysis System

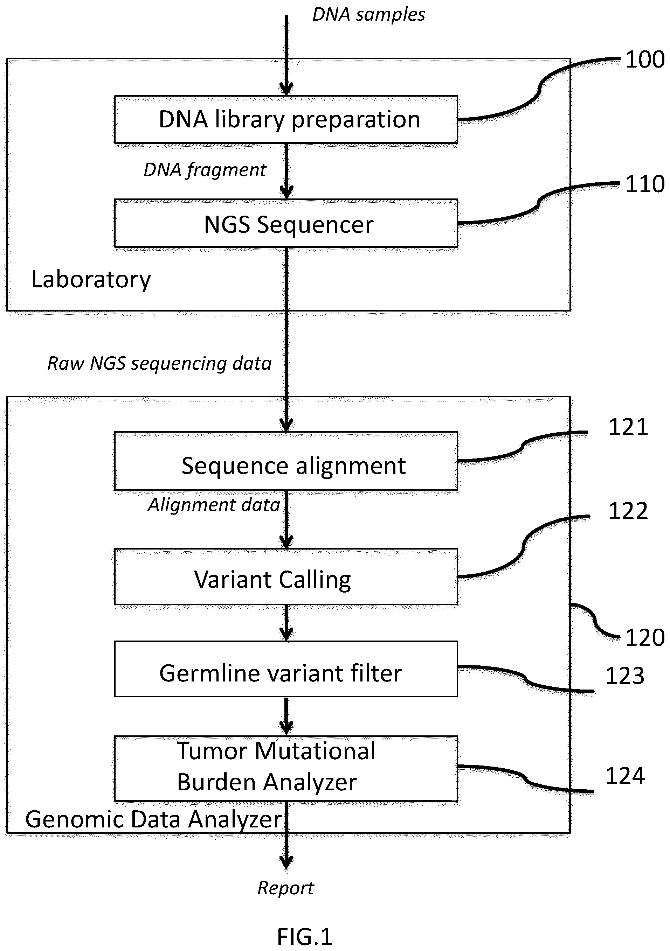

An exemplary genomic analysis system and workflow will now be described in further detail with reference to FIG.1. As will be apparent to those skilled in the art of DNA analysis, a genomic analysis workflow comprises preliminary experimental steps to be conducted in a laboratory (also known as the "wet lab") to produce DNA analysis data, such as raw sequencing reads in a next-generation sequencing workflow, as well as subsequent data processing steps to be conducted on the DNA analysis data to further identify information of interest to the end users, such as the detailed identification of DNA variants and related annotations, with a bioinformatics system (also known as the "dry lab"). Depending on the actual application, laboratory setup and bioinformatics platforms, various embodiments of a DNA analysis workflow are possible. FIG.1 describes an example of an NGS system comprising a wet lab system wherein DNA samples are first experimentally prepared with a DNA library preparation protocol 100 which may produce, adapt for sequencing, and amplify DNA fragments to facilitate the processing by an NGS sequencer 110. In a next generation sequencing workflow, the resulting DNA analysis data may be produced as a data file of raw sequencing reads in the FASTQ format. The workflow may further comprise a dry lab Genomic Data Analyzer system 120 which is configured to take, as input, the raw sequencing reads for a pool of DNA samples prepared according to the proposed methods and apply a series of data processing steps to characterize certain genomic features of the input samples. An exemplary Genomic Data Analyzer system 120 is the SOPHiA GENETICS DDM™ platform as already used by more than 1000 hospitals worldwide to automatically identify and characterize genomic variants and report them to the end user, but other systems may be used as well. Different detailed possible embodiments of data processing steps as may be applied by the Genomic Data Analyzer system 120 for genomic variant analysis are described for instance in the international PCT patent application WO2017/220508, but other embodiments are also possible.

As illustrated in FIG.1, the Genomic Data Analyzer 120 may comprise a sequence alignment module 121, which compares the raw NGS sequencing data to a reference genome, for instance the human genome in medical applications, or an animal genome in veterinary applications. In a conventional Genomic Data Analyzer system, the resulting alignment data may be further filtered and analyzed by a variant calling module 122 to retrieve variant information such as SNP and INDEL polymorphisms. The variant calling module 122 may be configured to execute different variant calling algorithms. The resulting detected variant information may then be output by the Genomic Data Analyzer module 120 as a genomic variant report for further processing by the end user, for instance with a visualization tool, and/or by a further variant annotation processing module (not represented). In a possible embodiment, the Genomic Data Analyzer system 120 may further comprise automated data processing modules such as a Tumor Mutational Burden (TMB) Analyzer module 124 configured to estimate the TMB value and/or determine a TMB-high or a TMB-low status, which may then be reported to the end user, for instance with a visualization tool, or to another downstream process (not represented). In another embodiment, the Genomic Data Analyzer system 120 may include a Germline variant filter 123 configured to filter likely germline variants and utilize such an output to calculate an associated TMB value and/or TMB status. Accordingly, the Germline variant filter 123 and TMB Analyzer 124 may be in informatic communication and may be adapted to function in tandem and/or to supplement each other. In one embodiment, the Germline variant filter 123 and the TMB Analyzer 124 may be independent modules. Yet, in another embodiment, the TMB Analyzer 124 may contain the Germline variant filter 123. Thus, for the purposes of this disclosure, actions executed by the TMB Analyzer 124 may, in some embodiments, be independently executed by the Germline variant filter 123. Conversely, actions disclosed as executed by the Germline variant filter 123 may be incorporated and/or executed by the TMB Analyzer 124.

Data processing workflow

The Genomic Data Analyzer 120 may process the sequencing data to produce a genomic data analysis report by employing and combining different data processing methods.

The sequence alignment module 121 may be configured to execute different alignment algorithms. Standard raw data alignment algorithms such as Bowtie2 or BWA that have been optimized for fast processing of numerous genomic data sequencing reads may be used, but other embodiments are also possible. The alignment results may be represented as one or several files in BAM or SAM format, as known to those skilled in the bioinformatics art, but other formats may also be used, for instance compressed formats or formats optimized for order-preserving encryption, depending on the genomic data analyzer 120 requirements for storage optimization and/or genomic data privacy enforcement.

The resulting alignment data may be further filtered and analyzed by a variant calling module 122 to retrieve variant information such as SNP and INDEL polymorphisms. The variant calling module 122 may be configured to execute different variant calling, variant annotation, variant prioritization and/or variant classification algorithms. The variant calling module 122 may produce a list of identified variants for the sample as a variant calling file in the VCF format or other suitable file format. Resulting detected variant information may then be output by the genomic data analyzer module 120 as a genomic variant report for further processing by the end user, for instance with a visualization tool, and/or by a further variant annotation processing module (not represented).

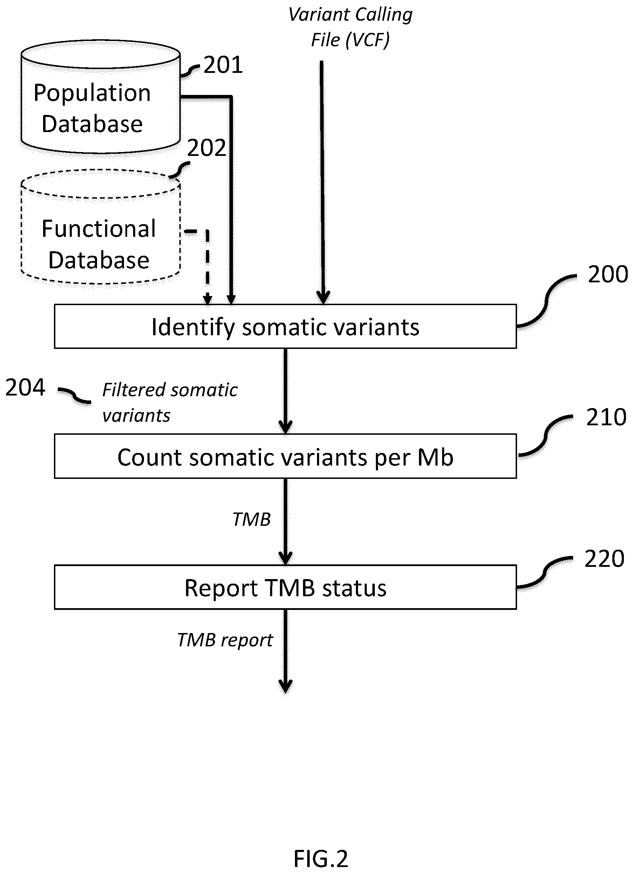

FIG. 2 shows a possible workflow for the automated TMB Analyzer module 124 to produce a TMB report from the analysis of a variant listing file for the patient sample such as a Variant Calling File in the VCF format in a bioinformatics workflow. In a possible embodiment, the TMB Analyzer module 124 may be adapted to identify 200 the somatic mutations from the variant listing by filtering out the germline variants from the list of variants identified from the variant calling module 122, to identify a list of germline variants and to retain only the variants which have not been identified as germline in a list of somatic variants to be counted for estimating the TMB value. In particular, in accordance with the TMB estimation methods from the prior art, for each variant in the VCF file, the TMB Analyzer module 124 may be adapted to identify from the population allele frequency as recorded in at least one population database 201 (e.g., GnomAD, 1000genome (g1000), Exome Variant Server, dbSNP, dbVar, Personalized Genome Project, ExAC, esp5400, or other suitable population genetics or germline variant databases), whether the variant is commonly found in the human population. In the latter case, the variant is most likely a germline variant which may then be included into the list of germline variants and may be removed from the list of possible somatic variants that are relevant for the TMB count. In accordance with the findings of the TMB Harmonization Project, in a possible embodiment, a variant may be filtered out as a germline variant if its pMAF (population minor allele frequency) is above a threshold of 0, 0.5%, or 1% in the population database reference. However, in various embodiments, the threshold may be set to any suitable degree. In another possible embodiment, a variant may be filtered out as a germline variant if its pMAF is outside a range of 0 to $10^{-6}$ in the population database reference. In another possible embodiment, a variant may be filtered out as a germline variant if there is at least one entry found for it in any of the searched population databases 201. In another possible embodiment, a variant may be filtered out as a germline variant if its pMAF is outside a range between 0 and 1/20000 in the population database reference. As will be apparent to those skilled in the art, other embodiments are also possible. In a possible embodiment, only variants found in coding regions are retained as possible somatic variants. In some embodiments, the sequence alignment module 121, the variant calling module 122, the germline variant filter 123, and/or the TMB Analyzer module 124 may be independent computerized components (i.e., stand-alone modules). In other embodiments, the sequence alignment module 121, the variant calling module 122, the germline variant filter 123, and/or the TMB Analyzer module 124 may be modules coexisting on the same system or device, for example, system 700, or electronic device 800.

In a possible further embodiment, for each of the remaining possible somatic variants, the TMB Analyzer module 124 and/or the Germline variant filter 123 may be adapted to identify variants recorded in at least one functional variant database or a functional database 202 (i.e., a cancer driven database), including, but not limited to, COSMIC, My Cancer Genome, cBioPortal, Personalized Cancer Therapy, ClinVar, Pediatric Cancer Genome Project, Intogen, or other suitable databases. In the latter case, it is most likely a somatic variant not associated with the tumor mutation load, which is then removed from the list of possible somatic variants that are relevant for the TMB count. For example, if a variant is present in one of the functional databases 202, then it is likely that such a variant may be a cancer-driven mutation and may be removed because TMB may be directed to measure the load of mutations in the tumor. In particular, a variant may be removed from the list of possible somatic variants if it is flagged as pathogenic or if it is a redundant variant in a functional variant database such as COSMIC. In a possible embodiment, a variant may be removed from the list of possible somatic variants if it is reported with at least 50 entries in, for example, a cancer-specific variant database. As will be apparent to those skilled in the art, other embodiments are also possible, for instance in accordance with the teachings of WO2017151524.

In some embodiments, the TMB Analyzer module 124 and/or the Germline variant filter 123 may be further adapted to determine the probability of a hidden germline status for a genomic series of possible somatic variants along each chromosome, using a statistical filter such as Hidden Markov Model (HMM) filter. In effect, the TMB Analyzer module 124 may be configured to determine the probability of a given variant identified in a somatic sample to be germline. Thus, the TMB Analyzer 124 may be utilized to compute a probability that a given variant is a germline variant for each putative somatic variant on each chromosome. The TMB Analyzer module 124 may then only retain and count as somatic variants those identified with a likely somatic state according to the HMM filter. In another embodiment, the HMM filter is configured to output the list of somatic variants. For example, instances where the HMM filter is configured to output the list of somatic variants may be utilized in conjunction with TMB estimation or other workflows requiring the identification of somatic variants from tumor only data.

In some embodiments, the TMB Analyzer module 124 may be further adapted to count 210 the number of somatic variants per unit of sequencing data length (e.g., by nucleotide), to estimate the TMB value for the patient sample. The TMB Analyzer module 124 may be further adapted to report 220 to the end user the TMB value estimate for the tumor sample. In a possible embodiment, the TMB Analyzer module 124 may produce a TMB value. In another possible embodiment, the TMB Analyzer module 124 may produce a TMB status, such as a negative status ("TMB-low"), a positive status ("TMB-high"), or an undetermined status. In a possible embodiment, the TMB status may be inferred based on the TMB value, for instance by applying a threshold, yet other embodiments are also possible. This report may then facilitate the selection of a medical treatment specifically targeting the analyzed tumor, including, but not limited to, immunotherapy treatment, immune checkpoint inhibitor (ICI) treatment, PD-L1 blockade treatment, or a CTLA4 blockade treatment.

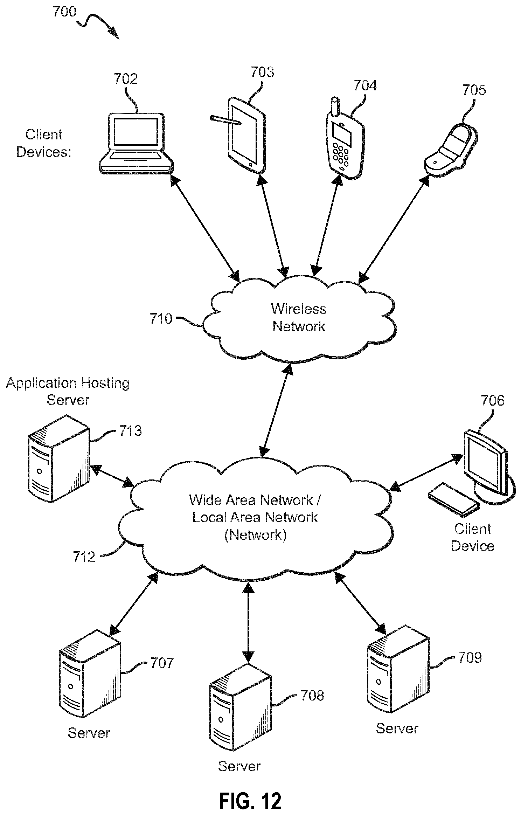

The Genomic Data Analyzer 120 may be a computer system or part of a computer system including a central processing unit (CPU, "processor" or "computer processor" herein), memory such as RAM and storage units such as a hard disk, and communication interfaces to communicate with other computer systems through a communication network, for instance the internet or a local network. Examples of genomic data analyzer computing systems, environments, and/or configurations include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, graphical processing units (GPU), and the like. In some embodiments, the computer system may comprise one or more computer servers, which are operational with numerous other general purpose or special purpose computing systems and may enable distributed computing, such as cloud computing, for instance in a genomic data farm. In some embodiments, the genomic data analyzer 120 may be integrated into a massively parallel system. In some embodiments, the genomic data analyzer 120 may be directly integrated into a next generation sequencing system. The specifications of the aforementioned computer systems and/or servers is described in further detail below in reference to FIGS. 12-13. Accordingly, the Genomic Data Analyzer 120 and/or other computerized components of the instant workflow may include the functionality and/or parts of system 700 and/or electronic device 800. Further, the method steps described herein may be executed by the system 700 and/or electronic device 800.

The Genomic Data Analyzer 120 computer system may be adapted in the general context of computer system-executable instructions, such as program modules, being executed by a computer system. Generally, program modules may include routines, programs, objects, components, logic, data structures, and so on that perform particular tasks or implement particular abstract data types. As is well known to those skilled in the art of computer programming, program modules may use native operating system and/or file system functions, standalone applications; browser or application plugins, applets, etc.; commercial or open-source libraries and/or library tools as may be programmed in Python, Biopython, C/C++, or other programming languages; custom scripts, such as Perl or Bioperl scripts.

Instructions may be executed in distributed cloud computing environments where tasks are performed by remote processing devices that are linked through a communications network. In a distributed cloud-computing environment, program modules may be located in both local and remote computer system storage media including memory storage devices.

It is thus understood that methods described herein are computer-implemented methods.

Hidden Markov Model

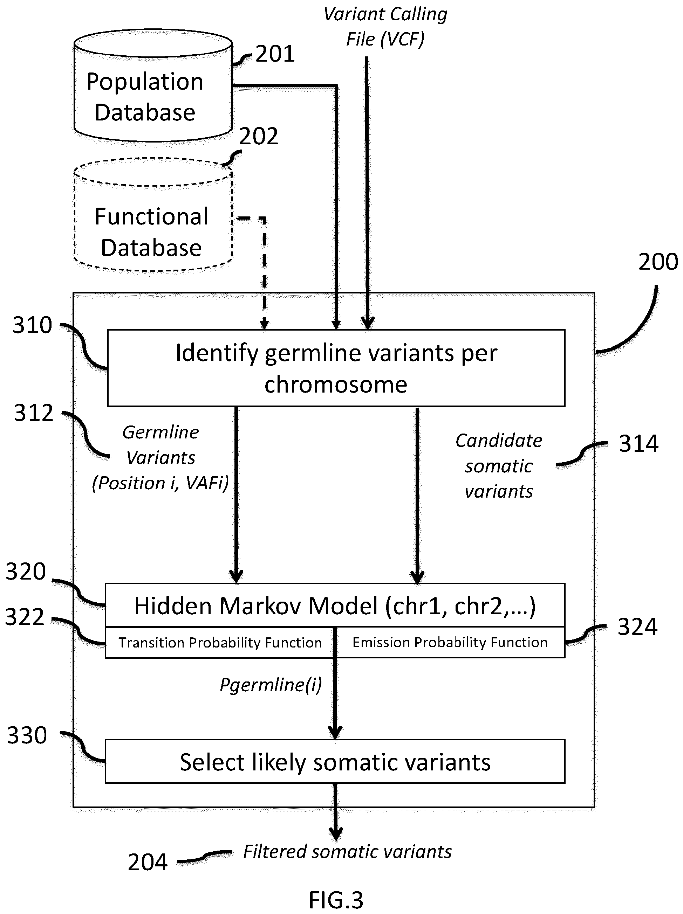

FIG. 3 schematically shows a possible workflow embodiment to improve the identification 200 of the somatic variants relevant for the TMB count by identifying which variants are likely somatic or likely germline variants 312 among a set of candidate somatic variants 314. The TMB Analyzer module 124 and/or the Germline variant filter 123 may be adapted to employ a Hidden Markov Model (HMM) 320 to estimate the probability that a candidate somatic variant is likely a germline variant, from the observation of the variant fractions (VF) in the list of germline variants along each chromosome. As shown in FIG. 3, the HMM 320 may be utilized to determine a probability that a particular variant is a germline variant. The resulting probability may next be utilized as, or in conjunction with, a filter to select likely somatic variants 330, further resulting in filtered somatic variants 204. In sum, the probability may be estimated utilizing the HMM 320, wherein, in turn, said probability may be used to filter.

The TMB Analyzer module 124 and/or the Germline variant filter 123 may, based on a population database 201 and/or a functional database 202 in accordance with any of the above-described methods, identify 310 the status of observed variants, for example, in the DNA obtained from a tumor sample from a patient, as either germline or candidate somatic variants and pre-classify said variants in a list of germline variants 312 and a list of candidate somatic variants 314, respectively.

To filter-out germline variants identified in the DNA from the tumor sample of the patient, not found in population database 201 and/or functional database 202 the TMB analyzer 124 can use a Hidden Markov Model filter 320, where the hidden state is the variant fraction of the variant at position $i$ (hidden variant fraction $VF_H(i)$) in the sample and the observation, also referred to as the emission state, is the variant fraction measured for variant at position $i$ (observed variant fraction, $VF_O(i)$) in the sample. Thus, in effect, the variant fraction measured for a variant at position $i$ may be measured based on sequencing of the DNA samples from the patient.

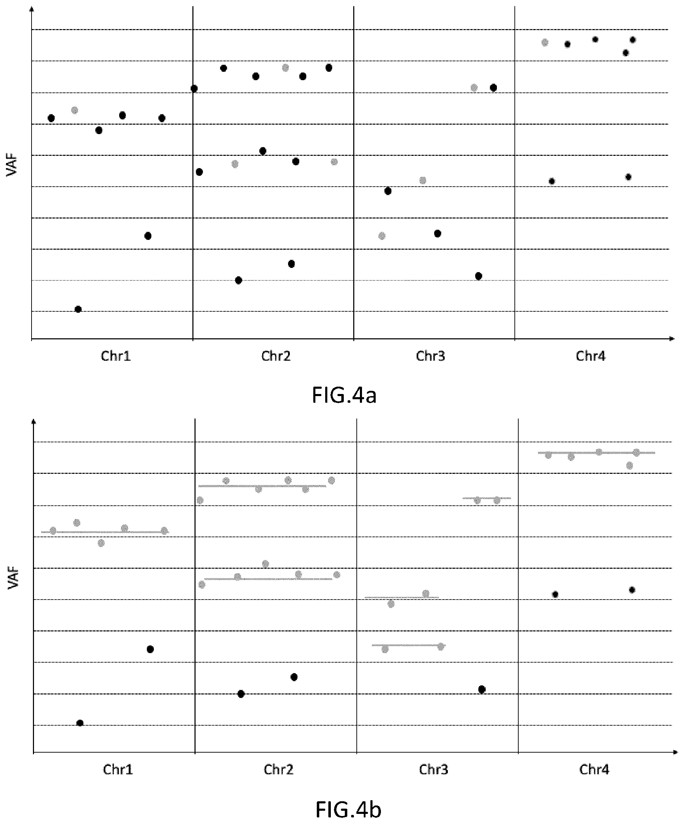

As non-limiting examples, and as schematically illustrated by FIG. 4A, the variants depicted in gray correspond to variants initially identified 310 as germline 312, while the variants depicted in black are the remaining candidate somatic variants 314. FIG. 4B illustrates the result of an embodiment of the proposed method, after filtering with an embodiment of the proposed HMM filter, to identify the most likely germline (i.e., newly marked in grey) versus the most likely somatic (i.e., retained in black) from the list of candidate somatic variants 314, according to the probability of a variant being germline for each candidate somatic variant, as estimated with the HMM implemented in the HMM filter 320.

In some embodiments, the hidden states are the discretized variant fractions $VF_H(i)$ for germline variants along a given genomic interval, as a non-limiting example, along each chromosome (i.e., as represented with dashed horizontal lines for the discretized values and light vertical lines for the chromosome separations in FIG. 4A and FIG. 4B). Indeed, the copy number of each individual chromosome may be independent from other chromosomes, so it is possible for the TMB Analyzer module 124 to separately process the genomic data for each chromosome genomic interval in the variant calling data file. In other words, since the copy number of each individual chromosome can be different, it is possible for the TMB analyzer module 124 to separately process the genomic data for each chromosome genomic interval in the variant calling data file. As will be apparent to those skilled in the art of bioinformatics, depending on its computing architecture design, for a given patient sample, the TMB Analyzer module 124 may select and identify 310 the lists of germline and candidate somatic variants along each chromosome serially. Further, the TMB Analyzer module 124 may select and identify the lists of germline and candidate somatic variants along each chromosome in parallel from the variant calling data VCF file (e.g., the VCF output file) in accordance with one or more reference database (e.g., a population database 201 or, optionally, a functional database 202). Thus, to clarify, the TMB analyzer module 124 may identify the lists of germline and candidate somatic variants in series, in parallel, and/or in a combination thereof. Various resolutions for the discretized hidden states $VF_H(i)$ values may also be used by the Hidden Markov Model 320. In an embodiment, the lower the number of states, the faster the computation, yet the less accurate the filter. In some embodiments, the number of hidden states of the HMM filter may be 9 $VF_H(i)$ value estimates (VF={10%, 20%,..., 90%}); 19 $VF_H(i)$ value estimates (VF=5%, 10%, 15%..., 95%); 99 $VF_H(i)$ value estimates (VF=1%, 2%, ..., 99%); 140 value estimates (VF=1/140%, 2/140%, 3/140%, ... 140/140%; more than 140 value estimates; 200 value estimates; or any suitable quantity of value estimates. In some embodiments, the number of observation states of the HMM filter may be 9 $VF_O(i)$ value estimates (VF=10%, 20%,..., 90%); 19 $VF_O(i)$ value estimates (VF=5%, 10%, 15%..., 95; 99 $VF_O(i)$ value estimates (VF=1%, 2%, ..., 99%); 80 (VF=1/80%, 2/80%, 3/80%, ...80/80%); or other suitable quantities. In an embodiment, as represented above, the value estimates may be determined by rounding to the nearest discretized fraction. However, in various embodiments, the value estimates may be determined, calculated, or otherwise generated via any suitable means.

In an embodiment, the probability of a given candidate somatic variant, with an observed variant fraction $VF_O(i)$ originating from the hidden state $VF_H(i)$, is determined by the **emission probability** function 324.

In some embodiments, the emission probability function is defined as:

$$P\big(VF_O(i)\big|VF_H(i)\big) = 0.9 \times \text{Beta}\big((\alpha, \beta); VF_O(i)\big) + 0.1 \times \text{Uniform}\big(0, 1\, ; VF_O(i)\big)$$

where the Beta distribution may be used to estimate the probability of $VF_O(i)$ given $VF_H(i)$ and may be defined by the parameters $\alpha$ and $\beta$, where $\alpha$ and $\beta$ may be defined as follows:

$$\alpha = 1 + \text{VF}_H(i);$$

and

$$\beta = 1 + D * (1 - \mathrm{VF_H}(i));$$

where **D** is the mean coverage depth observed for all variants in the sample obtained from the sample's corresponding VCF file. However, other suitable functions may be utilized for the emission probability function.

To account for outliers when estimating the probability of a given candidate somatic variant with an observed variant fraction $VF_O(i)$ originating from the hidden state $VF_H(i)$, a uniform distribution may be added to the Beta distribution (i.e., with a weight of 10%). However, a uniform distribution may be added comprising any suitable weight.

The probability of the variant fraction at a given genomic position, $i$ - 1 ($VF_H(i$ - 1)), to transition to a variant fraction $VF_H(i)$ at position i may be given by the **transition probability.**

In some embodiments, the variant fraction $VF_H(i)$, in a list of germline variants ordered according to their genomic position, is conditioned by the variant fraction of the previous variant in the list $VF_H(i$ - 1) according to the transition probability. The transition probability function 322 may be represented as:

$$P(VF_H(i)|VF_H(i-1)) = \begin{cases} 1 - p_{Switch} \text{ if } VF_H(i) = VF_H(i-1) \\ \qquad p_{Switch} \text{ otherwise} \end{cases}$$

where $p_{switch}$ represents the probability that the two variant fractions $VF_H(i$ - 1) and $VF_H(i)$ belong to different hidden states, for example, characterizing different copy number changes (or other genomic aberrations altering variant fraction). In an embodiment, the subsequent variants, $i$ and $i$ - 1, may be the closest genomic variants in genomic space. In a possible embodiment, $p_{switch}$ may be set in a range from 1e-3 to 1e-6 (i.e., a value of 2e-4), however, $p_{switch}$ may be set in any suitable range. In one embodiment, the HMM model employs a transition probability $p_{switch}$ representing the probability that the two subsequent variants, $i$ - 1 and $i$, along a chromosome, with variant fractions, $VF_H(i$ - 1) and $VF_H(i)$, are associated to the same hidden state. $p_{switch}$ may be chosen in a range from 1e-3 to 1e-6, for example, around a value of 2e-4.

Given a transition and emission probability, a set of $VF_H$, and an initial probability, the TMB Analyzer module 124 and/or HMM 320 may then be adapted to employ an algorithm configured to infer the posterior probability distribution of the hidden states at each genomic location $i$ given the $VF_O$. The algorithm may be a forward-backward algorithm (Collins, Encyclopedia of Biometrics 2009) adapted to compute "forwards" and "backwards" probabilities in the Hidden Markov Model and to infer the posterior probability distribution of the hidden states at each genomic location $i$ given the $VF_O$. In an embodiment, the transition and emission probability may be used by a forward-backward model (or similar suitable model) to infer the probability of a given hidden state (i.e., which may be a proxy for the most likely variant fraction in the sample) given the observed VF measured in the NGS file and reported typically in the VCF and the model parameters.

For each variant $j$ in the list of candidate somatic variants 314, the likelihood that the candidate somatic variant is of germline origin **Pgermline(j)** may be obtained based on $VF_O(j)$ and the posterior probability for $VF_H(i)$, where $i$ is the closest germline variant 312 in the genome to $j$.

The TMB Analyzer module 124 may then be further adapted to classify whether the candidate somatic variants 314 are likely germline variants according to **Pgermline(j)** obtained from the Hidden Markov Model 320.

In a possible embodiment, a threshold **(PgermlineThreshHMM)** may then be applied to each **Pgermline(j)** value to classify whether the corresponding variant candidate is germline. In a possible embodiment, the threshold **(PgermlineThreshHMM)** may be chosen around 1e-2, 1e-3, or 1e-4. However, the threshold may be chosen around any suitable quantity. The TMB Analyzer module 124 may further be configured to filter out the variants classified as likely germline variants from the list of candidate somatic variants 314 to select 330 only the likely somatic variants prior to counting 210 them per Mb to estimate the TMB value. In an alternate embodiment, the TMB Analyzer module 124 may be adapted to estimate, from the list of **Pgermline(j)** values, a probability distribution for the TMB value.

HMM Input Pre-Processing

In some embodiments, the proposed HMM 320 may be further optimized to improve its accuracy and/or the computational efficiency of the TMB Analyzer module 124. For example, without folding, the observations (i.e., the VAFs) may cover the range from 0 to 100%. This may need to be discretized to support such implementation. By folding VF there may be fewer states that need to be discretized (i.e., only 0-50%). This may allow for using the same number of discrete states to increase accuracy, and, if the number of states is reduced by 2, to preserve accuracy while improving computation efficiency.

In a possible embodiment, for each chromosome, the HMM 320 may take as input the list of heterozygous germline variants along that chromosome by retaining only those observed with a variant fraction with a predetermined range (e.g., above 5% and below 90%). As will be apparent to those skilled in the art of bioinformatics, copy number duplication and deletion events (CNV events) are expected to create contiguous genome intervals with variants at similar variant fraction data for heterozygous variants that are symmetric around 50%. In an embodiment, the observed variant fraction used as data input to the HMM model may be further "folded" (i.e., bringing VFs above 50% together with their symmetric counterpart bellow 50%) using the function defined as:

$$\mathrm{fold}(VF) = \begin{cases} VF & \text{if } VF < 1/2 \\ 1 - VF & \text{otherwise} \end{cases}$$

Thus, if the VF is above 50%, it may be replaced by observed state value 1-VF. However, the fold function may induce replacement of the VF to any suitable value when compared to any suitable predetermined threshold (i.e., ½).

In an embodiment, multi-nucleotide variants (for example, defined as two or more nearby variants belonging to the same haplotype of an individual) may be removed from the set of candidate variants prior to input to the HMM 320.

In the HMM 320, the transition probability may not depend on the genomic distance between variants. In order to overcome possible resulting biases (for example, a putative somatic variants might be erroneously filtered if located in between two distant germline variants), in further embodiment, the list of germline variants may be enriched with artificial data prior to the HMM input, as a non-limiting example, by adding non-observed data points on a regular grid along the chromosome genomic coordinates, which enable the hidden states to decorrelate when variants are far apart (e.g., located more than 10MB apart on the same chromosome). The emission probability for non-observed data points is set to a uniform distribution over the observed states, for all hidden states. In an embodiment, the genomic coordinates resolution for the grid of non-observed data points may be set to roughly 1MB, 2MB, 3MB, 4MB, 5MB, or, more generally, to a resolution chosen such that there are several grid points between the majority of observed variants. As a non-limiting example, a regular grid may refer to adding non-observed data points at regular genomic intervals, for example every 1 MB or every 2MB.

In an embodiment, only variants with a depth above or equal to 50x (or any suitable coverage depth) may be retained as candidate variants input to the HMM 320. The mean depth, D, of variants in the samples may also be rescaled to better adapt the binomial model to the actual noise of the samples data. In an embodiment, the mean depth, D, may be rescaled by a factor of ½, ¼, $1/_6$, or other suitable fraction, such that the posterior distribution computed by the HMM sufficiently covers the data. As a non-limiting example, the mean coverage depth D may be rescaled by multiplying the mean coverage depth D by a factor chosen in a range of 1/2 to 1.

As will be apparent to those skilled in the art of bioinformatics workflows, the above listed pre-processing filtering embodiments, as well as the prior art pre-processing embodiments of selecting candidate variants in accordance with the corresponding references in one or more reference population database 201 and/or in one or more functional database 202, may be implemented alone or in combination or in any order.

Example 1 - Comparison of TMB results obtained using TMB analyzer 124 with methods described herein and alternative methods

In one experiment, DNA extracted from 88 FFPE tumor samples may be sequenced using an Illumina NextSeq sequencer according to Illumina TruSight™ Oncology 500 panel instructions for use. The sequencing data obtained from these experiments may be analyzed to estimate TMB for each sample using two different implementations. In one implementation (implementation A), data may be analyzed as part of the SOPHiA DDM™ for TSO500 fully integrated bioinformatic Genomic Data Analyzer workflow 120 (FASTQ to Report) for Illumina TruSight™ Oncology 500 panel 100. As a non-limiting example, analysis may involve the steps of: quality control of sequencing data; alignment of sequencing data to the reference human genome (i.e., hg19); Single Nucleotide Variant (SNV) and small insertions/deletions (INDELS) variant calling; and outputting, using a variant calling file (VCF), the genomic location, sequence, and variant fraction for all detected variants.

The presence of the variants found in each tumor sample may be investigated in the g1000, esp5400, ExAC and GnomAD databases 201. Variants found in the sample and in any of these databases at a pVAF above 1/20000 may be classified as being germline 312. The remaining variants may be classified as candidate somatic variants 314. For each sample, candidate somatic variants 314 may be considered within the TruSight™ Oncology 500 panel targeted regions with VF between 5% and 90% for the TMB estimation using the TMB Analyzer 124. The HMM filter 320 used by the TMB Analyzer 124 in implementation A may be parametrized using a transition probability $p_{switch}$ of 2e-4, the mean coverage depth $\boldsymbol{D}$ may be rescaled by multiplying it by ¼, the number of hidden states may be set to 140, and a grid size of 2Mb may be used. In an embodiment, a variant is considered as germline if Pgermline exceeded a threshold of 1e-3 and is not considered as somatic if the number of occurrences in the COSMIC database exceed 50.

In a second implementation (implementation B), the TMB may be estimated using proximity filters, as implemented in the Illumina TruSight Oncology 500 Local App and according to instructions for use.

In such an embodiment, the relationship between TMB estimates for the 88 samples obtained using implementation A and Implementation B may be highly correlated ($R^2$ = 0.997).

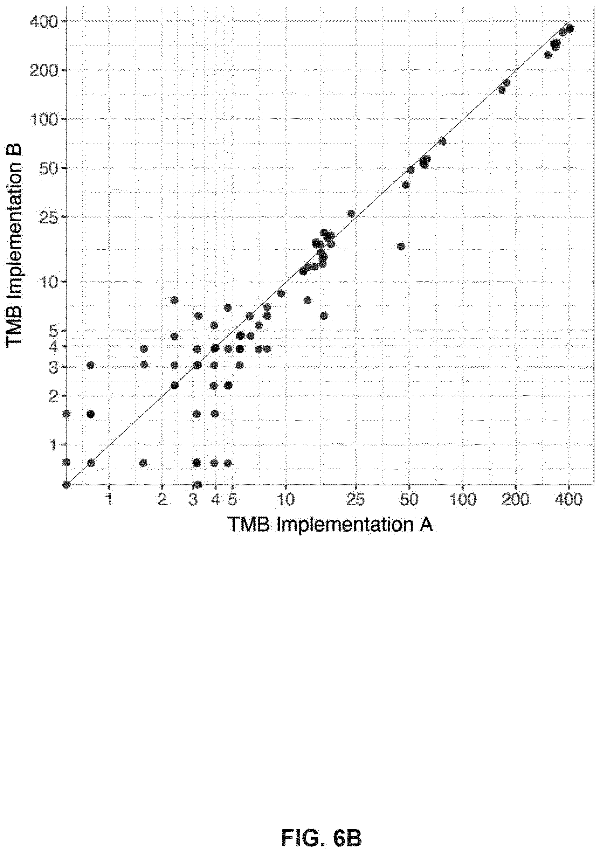

FIG. 6B shows the relationship between TMB results obtained using an embodiment of the method in the proposed implementation (implementation one) and the method used as reference (implementation two).

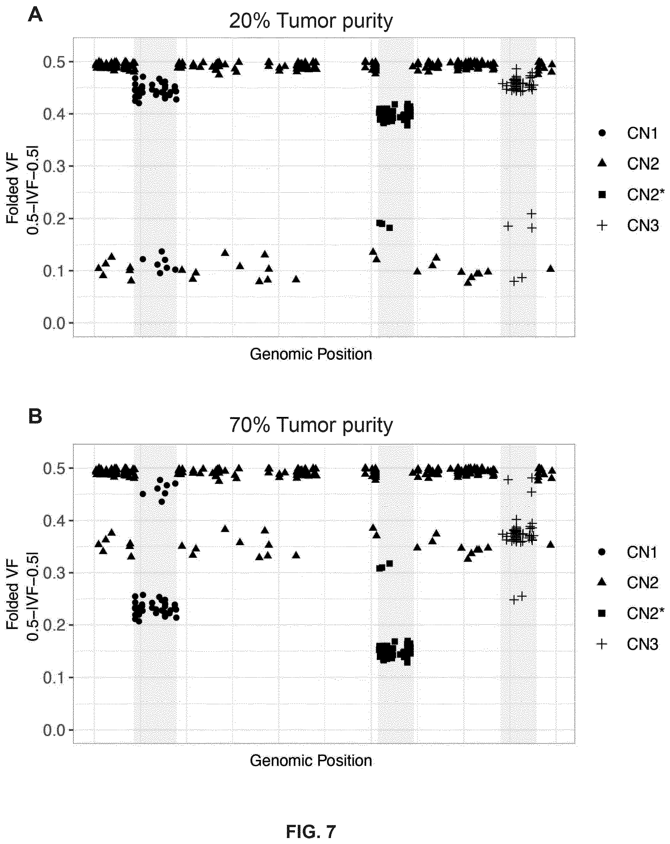

FIG. 7 shows the germline variants used as input for the HMM model (gray points), and for the remaining candidate somatic variants, in black, the output of the HMM filter classified as either somatic variants (black triangles) or germline variants (black points). The line contours represent the posterior distribution of the inferred VF of the germlines, which is separately calculated by the HMM for each chromosome.

<u>Example 2 - Evaluation of workflow for detection of somatic variants and TMB estimation from tumor only data</u>

### *Tumor DNA sequence data*

The impact of tumor content and copy number variation in the ability of different TMB analyzer implementations may be modeled to distinguish somatic from germline variants in the absence of a sequence for a matching normal sample. Chromosome 1 human reference sequence may be considered to simulate tumor genome data as following:

1) Common variants may be considered, defined as a variant present in GnomAD with a pVAF>1/20000, in exonic regions to simulate germline variants. The simulated sequence common variant may be sampled and added to according to their population variant allele frequencies. Sampling may be done separately for each of the two alleles. Following this procedure, a total of 551 'germline' variants with variant fraction of 0.5 or 1.0 may be added to the simulated sequence.

2) To simulate germline variants absent from allele frequency databases, 45 variants not present in GnomAD may be introduced. The variant fraction may be 0.5 for 40 of the variants and may be 1 for 5 of the variants.

3) To simulate DNA sequences obtained from samples with different tumor contents, variants may be introduced in 44 random exonic locations with variant fractions expected for samples with 20% and 70% tumor purities : $VF_{somatic} =$

$$TumorPurity * \frac{(as1+as2)}{2}$$

, where $as$1 and $as$2 represent presence/absence of mutation in the somatic alleles 1 and 2 at a given genomic coordinate, respectively.

4) To simulate the presence of factors that distort the measured VF from the sample VF a residual noise may be added to all simulated variants assuming a normal distribution. The residual noise $\sigma$ may be 0.01 VF for germline and 0.015 VF for somatic variants.

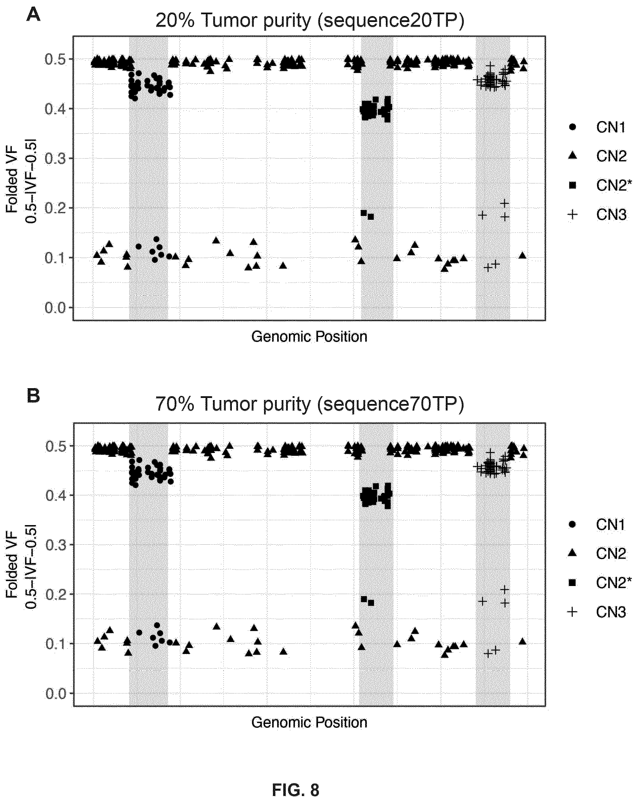

5) To simulate the impact of copy number loss and gain, the following may be simulated, for example, as shown in FIG. 8,

a. The loss of an allele (CN1)
b. a copy neutral loss of heterozygosity (CN2*)
c. and an allele duplication (CN3)

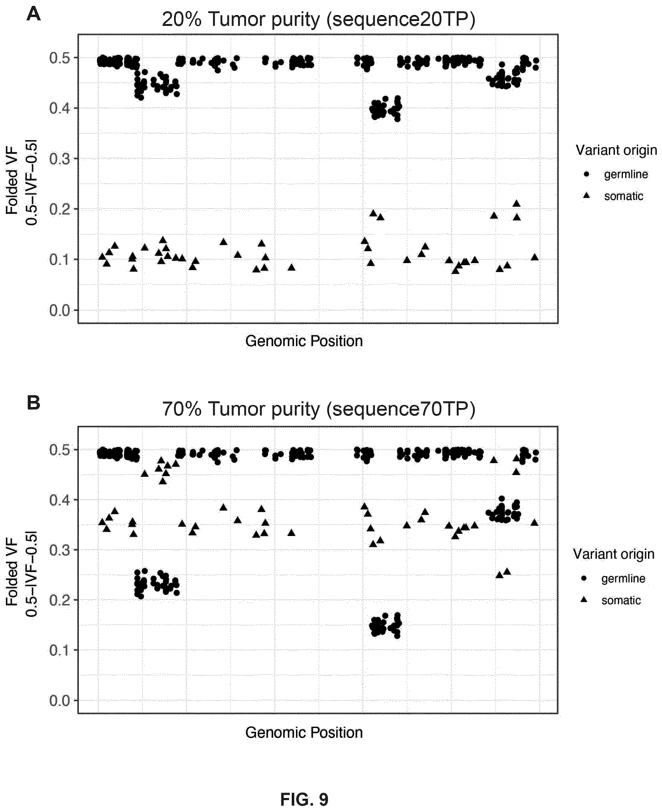

The resulting distribution of the VFs of simulated variants following this implementation along genomic coordinates for germline and somatic variants may be represented in FIG. 9.

### *Detection of somatic variants using tumor only data in simulated sequences*

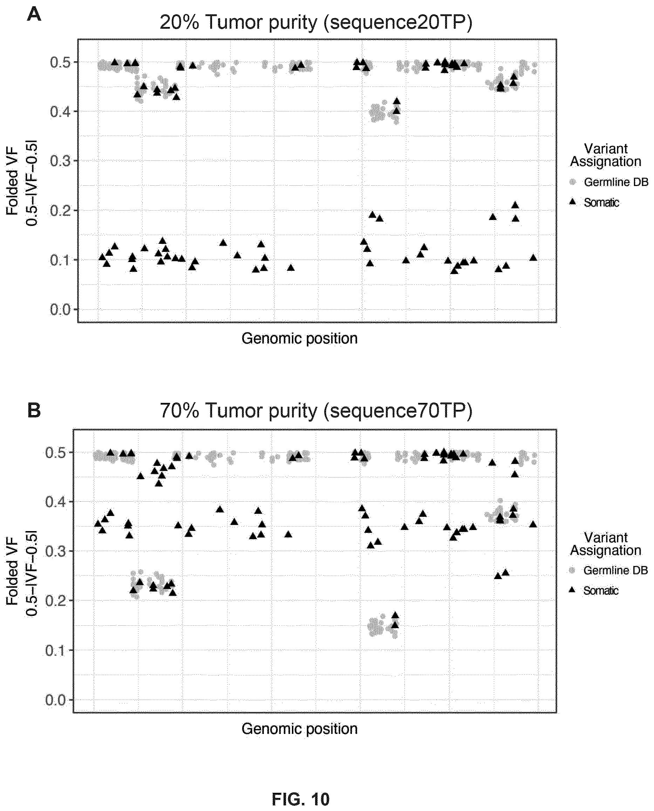

The VCF may be considered for simulated sequence 20% and 70% tumor purity, hereafter sequence20TP and sequence70TP. For variants with VF between 5% and 90%, the presence of the variants found in the simulated sequences in the g1000, esp5400, ExAC and GnomAD databases 201 may be evaluated. Variants found in the sample and in any of these databases at a pVAF above 1/20000 may be annotated as being germline 312. The remaining variants may be classified as candidate somatic variants 314. The number of somatic variants identified (FIG. 10) present on coding sequences and the total coding sequence length of 3.32 megabases covered by the simulations to estimate TMB may be considered. Table XX row "Database Only" may summarize the number of variants in each class and the TMB, estimated for sequence20TP and sequence70TP.

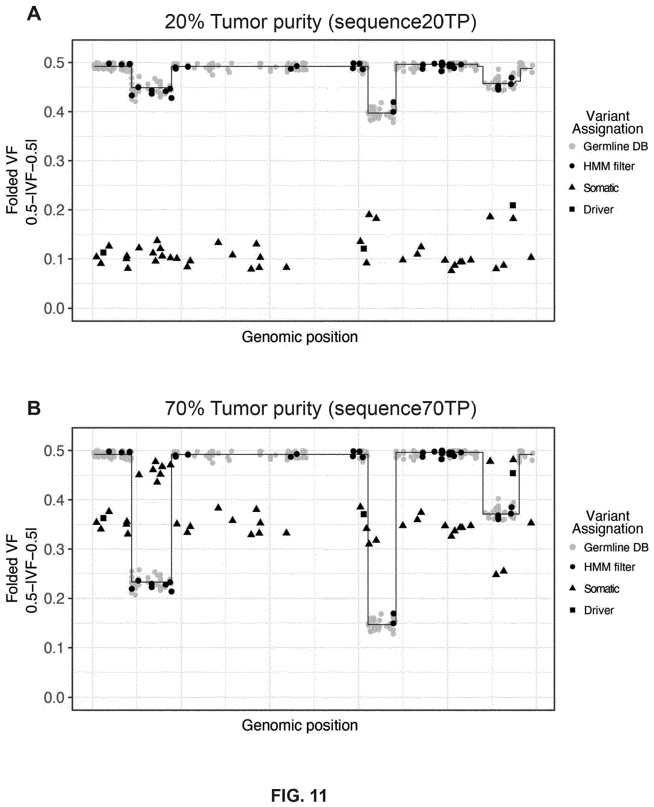

Next, the HMM filter may be implemented using as input 381 germline 312 and 44 candidate somatic variant 314 with VF between 5% and 90%. The HMM filter may be parametrized using a transition probability $p_{switch}$ of 5e-4, the number of hidden states may be set to 140, and a grid size of 1.5Mb may be used. Further, the variant may be considered as germline if Pgermline exceeds a threshold of 1e-3 (FIG. 11). Three somatic variants may be excluded that occurred at least 50 times in the functional database COSMIC 202. Table XX row "Database+HMM filter" may summarize the number of variants in each class and the TMB, obtained for sequence20TP and sequence70TP in this manner.

Table XX may indicate the numbers of germline and filtered somatic variants classified by population allele frequency databases or the same method augmented with HMM filter and a cancer-specific database to identify driver mutations. Driver mutations may be indicated in parenthesis. For the purposes of this disclosure, CDS may refer to "Coding Sequence."

TABLE XX

| | 20% tumor purity | | | | 70% tumor purity | | | |
|---|---|---|---|---|---|---|---|---|
| | Germline variants | Filtered Somatic variants (driver) | Somatic variants on CDS (driver) | TMB [Mut./Mb] | Germline variants | Somatic variants (driver) | Somatic variants on CDS (driver) | TMB [Mut./Mb] |
| Ground truth | 381 | 41 (3) | 26 (3) | 7.83 | 381 | 41 (3) | 26 (3) | 7.83 |
| Databases only | 341 | 84 | 63 | 18.98 | 341 | 84 | 63 | 18.98 |
| Databases+ HMM filter | 381 | 41 (3) | 26 (3) | 7.83 | 381 | 41 (3) | 26 (3) | 7.83 |

Further Advantages And Benefits Of The Proposed Methods

As will be apparent to those skilled in the art, the proposed HMM based approach, calculated independently for each chromosome and using the pre-processed germline variants as input to the HMM model, may enable the system to balance the prior transition probability with the presence/absence of CNVs in the data. Accordingly, this favors a more accurate germline VAF prediction for each sample.

Diagnostic Methods / Treatment Selection

In an embodiment, the TMB status or related values may be obtained by the methods described herein, wherein the TMB status or related values may be calibrated. For example, the TMB status or related values may be calibrated via estimation of a calibration curve having related predication limits to quantify the mean relationship between WES and panel TMB assay values. Further, the calibration may take into account variability of the calibration curve. In yet a further embodiment, uncertainty and confidence levels may be generated and correlated to accompany the corresponding calibrated TMB values.

In one embodiment, provided is a method for selecting a cancer patient for immunotherapy treatment comprising a step of determining a tumor mutational burden (TMB) value in the cancer patient tumor sample with any of the methods described herein.

In one embodiment, provided is a method for treatment selection for a cancer patient comprising a step of determining a tumor mutational burden (TMB) value in the cancer patient tumor sample with any of the methods described herein.

It is understood that a cancer patient with a TMB-high status may benefit from immunotherapy treatment. The TMB-high status may be determined with any of the methods described herein.

The immunotherapy treatment may include treatments including immune checkpoint inhibitors (ICI), for instance, a PD-L1 blockade treatment or a CTLA4 blockade treatment, for example, pembrolizumab, embrolizumab, ipilimumab, nivolumab, atezolizumab, and other suitable alternatives.

According to one aspect, a sample may be a patient sample and may be represented in a form of tissue, fresh frozen tissue (FFT), blood or any other suitable bodily fluid, or cytological specimens/preparation (FFPE, smears), and the like. According to one aspect, a sample is a patient tumor sample, e.g., a Formalin-Fixed Paraffin-Embedded (FFPE) sample.

In another embodiment, patients may be suffering from a cancer, including, but not limited to, lung cancer, melanoma, prostate cancer, bladder cancer, breast cancer, colorectal cancer, and the like.

According to one embodiment, provided is a method of diagnosing a cancer type having TMB-high status or TMB-low status according to the methods described herein.

Technical Structure Embodiments

The components, procedures, and steps described above may be executed, performed, or embodied by system 700, electronic device 800, or parts thereof, as described below and shown in FIGS. 12-13.

FIG. 12 illustrates components of one embodiment of an environment in which the invention may be practiced. Not all of the components may be required to practice the invention, and variations in the arrangement and type of the components may be made. As shown, the system 700 includes one or more Local Area Networks ("LANs")/Wide Area Networks ("WANs") 712, one or more wireless networks 710, one or more wired or wireless client devices 706, mobile or other wireless client devices 702-705, servers 707-709, and may include or communicate with one or more data stores or databases. Various of the client devices 702-706 may include, for example, desktop computers, laptop computers, set top boxes, tablets, cell phones, smart phones, smart speakers, wearable devices (such as the Apple Watch) and the like. Servers 707-709 can include, for example, one or more application servers, content servers, search servers, and the like. FIG. 12 also illustrates application hosting server 713.

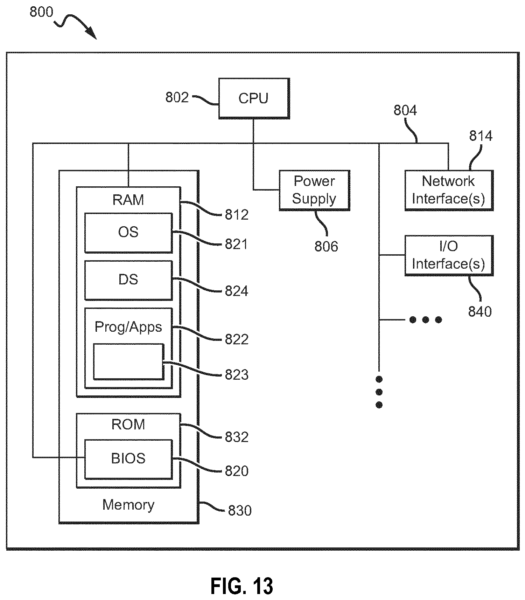

FIG. 13 illustrates a block diagram of an electronic device 800 that can implement one or more aspects of an apparatus, system and method for validating and correcting user information (the "Engine") according to one embodiment of the invention. Instances of the electronic device 800 may include servers, e.g., servers 707-709, and client devices, e.g., client devices 702-706. In general, the electronic device 800 can include a processor/CPU 802, memory 830, a power supply 806, and input/output (I/O) components/devices 840, e.g., microphones, speakers, displays, touchscreens, keyboards, mice, keypads, microscopes, GPS components, cameras, heart rate sensors, light sensors, accelerometers, targeted biometric sensors, etc., which may be operable, for example, to provide graphical user interfaces or text user interfaces.

A user may provide input via a touchscreen of an electronic device 800. A touchscreen may determine whether a user is providing input by, for example, determining whether the user is touching the touchscreen with a part of the user's body such as his or her fingers. The electronic device 800 can also include a communications bus 804 that connects the aforementioned elements of the electronic device 800. Network interfaces 814 can include a receiver and a transmitter (or transceiver), and one or more antennas for wireless communications.

The processor 802 can include one or more of any type of processing device, e.g., a Central Processing Unit (CPU), and a Graphics Processing Unit (GPU). Also, for example, the processor can be central processing logic, or other logic, may include hardware, firmware, software, or combinations thereof, to perform one or more functions or actions, or to cause one or more functions or actions from one or more other components. Also, based on a desired application or need, central processing logic, or other logic, may include, for example, a software-controlled microprocessor, discrete logic, e.g., an Application Specific Integrated Circuit (ASIC), a programmable/programmed logic device, memory device containing instructions, etc., or combinatorial logic embodied in hardware. Furthermore, logic may also be fully embodied as software.

The memory 830, which can include Random Access Memory (RAM) 812 and Read Only Memory (ROM) 832, can be enabled by one or more of any type of memory device, e.g., a primary (directly accessible by the CPU) or secondary (indirectly accessible by the CPU) storage device (e.g., flash memory, magnetic disk, optical disk, and the like). The RAM can include an operating system 821, data storage 824, which may include one or more databases, and programs and/or applications 822, which can include, for example, software aspects of the program 823. The ROM 832 can also include Basic Input/Output System (BIOS) 820 of the electronic device.

Software aspects of the program 823 are intended to broadly include or represent all programming, applications, algorithms, models, software and other tools necessary to implement or facilitate methods and systems according to embodiments of the invention. The elements may exist on a single computer or be distributed among multiple computers, servers, devices or entities.

The power supply 806 contains one or more power components and facilitates supply and management of power to the electronic device 800.

The input/output components, including Input/Output (I/O) interfaces 840, can include, for example, any interfaces for facilitating communication between any components of the electronic device 800, components of external devices (e.g., components of other devices of the network or system 700), and end users. For example, such components can include a network card that may be an integration of a receiver, a transmitter, a transceiver, and one or more input/output interfaces. A network card, for example, can facilitate wired or wireless communication with other devices of a network. In cases of wireless communication, an antenna can facilitate such communication. Also, some of the input/output interfaces 840 and the bus 804 can facilitate communication between components of the electronic device 800, and in an example can ease processing performed by the processor 802.

Where the electronic device 800 is a server, it can include a computing device that can be capable of sending or receiving signals, e.g., via a wired or wireless network, or may be capable of processing or storing signals, e.g., in memory as physical memory states. The server may be an application server that includes a configuration to provide one or more applications, e.g., aspects of the Engine, via a network to another device. Also, an application server may, for example, host a web site that can provide a user interface for administration of example aspects of the Engine.

Any computing device capable of sending, receiving, and processing data over a wired and/or a wireless network may act as a server, such as in facilitating aspects of implementations of the Engine. Thus, devices acting as a server may include devices such as dedicated rack-mounted servers, desktop computers, laptop computers, set top boxes, integrated devices combining one or more of the preceding devices, and the like.

Servers may vary widely in configuration and capabilities, but they generally include one or more central processing units, memory, mass data storage, a power supply, wired or wireless network interfaces, input/output interfaces, and an operating system such as Windows Server, Mac OS X, Unix, Linux, FreeBSD, and the like.

A server may include, for example, a device that is configured, or includes a configuration, to provide data or content via one or more networks to another device, such as in facilitating aspects of an example apparatus, system and method of the Engine. One or more servers may, for example, be used in hosting a Web site, such as the web site www.microsoft.com. One or more servers may host a variety of sites, such as, for example, business sites, informational sites, social networking sites, educational sites, wikis, financial sites, government sites, personal sites, and the like.

Servers may also, for example, provide a variety of services, such as Web services, third-party services, audio services, video services, email services, HTTP or HTTPS services, Instant Messaging (IM) services, Short Message Service (SMS) services, Multimedia Messaging Service (MMS) services, File Transfer Protocol (FTP) services, Voice Over IP (VOIP) services, calendaring services, phone services, and the like, all of which may work in conjunction with example aspects of an example systems and methods for the apparatus, system and method embodying the Engine. Content may include, for example, text, images, audio, video, and the like.

In example aspects of the apparatus, system and method embodying the Engine, client devices may include, for example, any computing device capable of sending and receiving data over a wired and/or a wireless network. Such client devices may include desktop computers as well as portable devices such as cellular telephones, smart phones, display pagers, Radio Frequency (RF) devices, Infrared (IR) devices, Personal Digital Assistants (PDAs), handheld computers, GPS-enabled devices tablet computers, sensor-equipped devices, laptop computers, set top boxes, wearable computers such as the Apple Watch and Fitbit, integrated devices combining one or more of the preceding devices, and the like.

Client devices such as client devices 702-706, as may be used in an example apparatus, system and method embodying the Engine, may range widely in terms of capabilities and features. For example, a cell phone, smart phone or tablet may have a numeric keypad and a few lines of monochrome Liquid-Crystal Display (LCD) display on which only text may be displayed. In another example, a Web-enabled client device may have a physical or virtual keyboard, data storage (such as flash memory or SD cards), accelerometers, gyroscopes, respiration sensors, body movement sensors, proximity sensors, motion sensors, ambient light sensors, moisture sensors, temperature sensors, compass, barometer, fingerprint sensor, face identification sensor using the camera, pulse sensors, heart rate variability (HRV) sensors, beats per minute (BPM) heart rate sensors, microphones (sound sensors), speakers, GPS or other location-aware capability, and a 2D or 3D touch-sensitive color screen on which both text and graphics may be displayed. In some embodiments multiple client devices may be used to collect a combination of data. For example, a smart phone may be used to collect movement data via an accelerometer and/or gyroscope and a smart watch (such as the Apple Watch) may be used to collect heart rate data. The multiple client devices (such as a smart phone and a smart watch) may be communicatively coupled.

Client devices, such as client devices 702-706, for example, as may be used in an example apparatus, system and method implementing the Engine, may run a variety of operating systems, including personal computer operating systems such as Windows, iOS or Linux, and mobile operating systems such as iOS, Android, Windows Mobile, and the like. Client devices may be used to run one or more applications that are configured to send or receive data from another computing device. Client applications may provide and receive textual content, multimedia information, and the like. Client applications may perform actions such as browsing webpages, using a web search engine, interacting with various apps stored on a smart phone, sending and receiving messages via email, SMS, or MMS, playing games (such as fantasy sports leagues), receiving advertising, watching locally stored or streamed video, or participating in social networks.

In example aspects of the apparatus, system and method implementing the Engine, one or more networks, such as networks 710 or 712, for example, may couple servers and client devices with other computing devices, including through wireless network to client devices. A network may be enabled to employ any form of computer readable media for communicating information from one electronic device to another. The computer readable media may be non-transitory. A network may include the Internet in addition to Local Area Networks (LANs), Wide Area Networks (WANs), direct connections, such as through a Universal Serial Bus (USB) port, other forms of computer-readable media (computer-readable memories), or any combination thereof. On an interconnected set of LANs, including those based on differing architectures and protocols, a router acts as a link between LANs, enabling data to be sent from one to another.

Communication links within LANs may include twisted wire pair or coaxial cable, while communication links between networks may utilize analog telephone lines, cable lines, optical lines, full or fractional dedicated digital lines including T1, T2, T3, and T4, Integrated Services Digital Networks (ISDNs), Digital Subscriber Lines (DSLs), wireless links including satellite links, optic fiber links, or other communications links known to those skilled in the art. Furthermore, remote computers and other related electronic devices could be remotely connected to either LANs or WANs via a modem and a telephone link.

A wireless network, such as wireless network 710, as in an example apparatus, system and method implementing the Engine, may couple devices with a network. A wireless network may employ stand-alone ad-hoc networks, mesh networks, Wireless LAN (WLAN) networks, cellular networks, and the like.

A wireless network may further include an autonomous system of terminals, gateways, routers, or the like connected by wireless radio links, or the like. These connectors may be configured to move freely and randomly and organize themselves arbitrarily, such that the topology of wireless network may change rapidly. A wireless network may further employ a plurality of access technologies including 2nd (2G), 3rd (3G), 4th (4G) generation, Long Term Evolution (LTE) radio access for cellular systems, WLAN, Wireless Router (WR) mesh, and the like. Access technologies such as 2G, 2.5G, 3G, 4G, and future access networks may enable wide area coverage for client devices, such as client devices with various degrees of mobility. For example, a wireless network may enable a radio connection through a radio network access technology such as Global System for Mobile communication (GSM), Universal Mobile Telecommunications System (UMTS), General Packet Radio Services (GPRS), Enhanced Data GSM Environment (EDGE), 3GPP Long Term Evolution (LTE), LTE Advanced, Wideband Code Division Multiple Access (WCDMA), Bluetooth, 802.11b/g/n, and the like. A wireless network may include virtually any wireless communication mechanism by which information may travel between client devices and another computing device, network, and the like.

Internet Protocol (IP) may be used for transmitting data communication packets over a network of participating digital communication networks, and may include protocols such as TCP/IP, UDP, DECnet, NetBEUI, IPX, Appletalk, and the like. Versions of the Internet Protocol include IPv4 and IPv6. The Internet includes local area networks (LANs), Wide Area Networks (WANs), wireless networks, and long-haul public networks that may allow packets to be communicated between the local area networks. The packets may be transmitted between nodes in the network to sites each of which has a unique local network address. A data communication packet may be sent through the Internet from a user site via an access node connected to the Internet. The packet may be forwarded through the network nodes to any target site connected to the network provided that the site address of the target site is included in a header of the packet. Each packet communicated over the Internet may be routed via a path determined by gateways and servers that switch the packet according to the target address and the availability of a network path to connect to the target site.

The header of the packet may include, for example, the source port (16 bits), destination port (16 bits), sequence number (32 bits), acknowledgement number (32 bits), data offset (4 bits), reserved (6 bits), checksum (16 bits), urgent pointer (16 bits), options (variable number of bits in multiple of 8 bits in length), padding (may be composed of all zeros and includes a number of bits such that the header ends on a 32 bit boundary). The number of bits for each of the above may also be higher or lower.

A "content delivery network" or "content distribution network" (CDN), as may be used in an example apparatus, system and method implementing the Engine, generally refers to a distributed computer system that comprises a collection of autonomous computers linked by a network or networks, together with the software, systems, protocols and techniques designed to facilitate various services, such as the storage, caching, or transmission of content, streaming media and applications on behalf of content providers. Such services may make use of ancillary technologies including, but not limited to, "cloud computing," distributed storage, DNS request handling, provisioning, data monitoring and reporting, content targeting, personalization, and business intelligence. A CDN may also enable an entity to operate and/or manage a third party's web site infrastructure, in whole or in part, on the third party's behalf.

A Peer-to-Peer (or P2P) computer network relies primarily on the computing power and bandwidth of the participants in the network rather than concentrating it in a given set of dedicated servers. P2P networks are typically used for connecting nodes via largely ad hoc connections. A pure peer-to-peer network does not have a notion of clients or servers, but only equal peer nodes that simultaneously function as both "clients" and "servers" to the other nodes on the network.

Embodiments of the present invention include apparatuses, systems, and methods implementing the Engine. Embodiments of the present invention may be implemented on one or more of client devices 702-706, which are communicatively coupled to servers including servers 707-709. Moreover, client devices 702-706 may be communicatively (wirelessly or wired) coupled to one another. In particular, software aspects of the Engine may be implemented in the program 823. The program 823 may be implemented on one or more client devices 702-706, one or more servers 707-709, and 713, or a combination of one or more client devices 702-706, and one or more servers 707-709 and 713.

Other embodiments and applications

While various embodiments have been described above, it should be understood that they have been presented by way of example and not limitation. It will be apparent to persons skilled in the relevant art(s) that various changes in form and detail can be made therein without departing from the scope. In fact, after reading the above description, it will be apparent to one skilled in the relevant art(s) how to implement alternative embodiments.

While exemplary embodiments and applications of the proposed methods have been described in relation with a HMM filter, it will be apparent to those skilled in the art of bioinformatics that various other embodiments are also possible, for instance using Gaussian processes, recurrent neural networks, or LSTM (Long Short Term Memory) networks. The filter may be adapted to estimate the probability of a variant $i$ to be somatic Psomatic($i$) rather than the probability of a variant $i$ to be germline Pgermline($i$), as, for example, Psomatic($i$) = 1-Pgermline($i$).

The invention of the present disclosure may be a computer-implemented method of filtering germline variants from a DNA sequence obtained from a tumor sample, the method comprising obtaining variant calling data for the tumor sample; identifying, in the variant calling data and in view of at least one population database, a list of germline variants for the tumor sample along each chromosome; identifying, in the variant calling data, a list of candidate somatic variants as remaining variants in the variant calling data that have not been identified in the list of germline variants; filtering out likely germline variants from the list of candidate somatic variants to retain only somatic variants, filtering out the likely germline variants from the list of candidate somatic variants to retain only the somatic variants comprising estimating, with a Hidden Markov Model (HMM) along each chromosome, a probability of each candidate somatic variant $i$ being a germline variant (Pgermline(i)); and determining, from the Pgermline(i), whether a candidate somatic variant i is germline or somatic, to retain only the likely somatic variants in the list of candidate somatic variants.

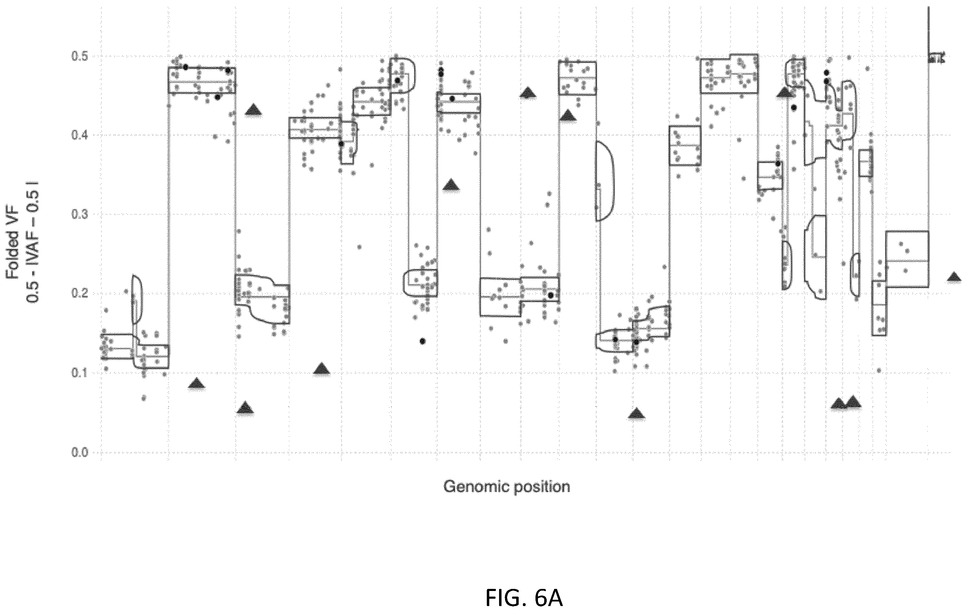

Referring to FIG. 6A, in one experiment, two different implementations of the Tumor Mutational Burden Analyzer module 124 have been tested as part of the SOPHiA DDM™ for TSO500 fully integrated bioinformatic Genomic Data Analyzer workflow 120 (FASTQ to Report) for an Illumina TruSight™ Oncology 500 panel 100. In the first implementation, used as the reference, the TMB calculation method of WO2020092591, based on proximity filters, may be used. In the second implementation, the proposed TMB calculation method, based on HMM filtering, may be used. For both implementations, the g1000, esp5400, ExAC and GnomAD population databases 201 may be searched for each variant which is annotated 310 as germline if a hit has been found in any of these databases. For clinical samples of the TSO500 panel, a high correlation ($R^2$=0.98) of TMB value estimation has been obtained between the two implementations. Thus, FIG. 6A shows the germline variants used as input for the HMM model (gray points), and for the remaining candidate somatic variants, in black, the output of the HMM filter classified as either somatic variants (black triangles) or germline variants (black points). The line contours represent the posterior distribution of the inferred VF of the germlines, which is separately calculated by the HMM for each chromosome.

The invention of the present disclosure may be a system or method configured for the identification of somatic variants from tumor-only data. Accordingly, such a system or method may utilize the steps and procedures described herein. For example, such a system or method may comprise many of the elements described herein, yet may more generally identify somatic variants without specifically inferring TMB.

Unless otherwise defined, all technical and scientific terms used herein have the same meaning as commonly understood by one of ordinary skill in the art to which this invention belongs. The terminology used in the description herein is for describing particular embodiments only and is not intended to be limiting. As used in the description and the appended claims, the singular forms "a," "an," and "the" are intended to include the plural forms as well, unless the context clearly indicates otherwise.

Unless indicated to the contrary, the numerical parameters set forth in the following specification and attached claims are approximations that may vary depending upon the desired properties sought to be obtained and thus may be modified by the term "about". At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

As will be apparent to those skilled in the art of digital data communications, the methods described herein may be indifferently applied to various data structures such as data files or data streams. The terms "data", "data set", "data structures", "data fields", "file", or "stream" may thus be used indifferently throughout this specification.

Although the detailed description above contains many specific details, these should not be construed as limiting the scope of the embodiments but as merely providing illustrations of some of several embodiments.

In addition, it should be understood that any figures which highlight the functionality and advantages are presented for example purposes only. The disclosed methods are sufficiently flexible and configurable such that they may be utilized in ways other than that shown.

**Claims**

1. A computer-implemented method of determining a tumor mutational burden (TMB) value in a tumor sample, the method comprising:

   obtaining variant calling data for the tumor sample;
   identifying, in the variant calling data and in view of at least one population database, a list of germline variants for the tumor sample along each chromosome,
   wherein each of the germline variants of the list of germline variants includes a population minor allele frequency (pMAF) above a threshold relative to the at least one population database;
   identifying, in the variant calling data, a list of candidate somatic variants,
   wherein the list of candidate somatic variants are the remaining variants in the variant calling data that have not been identified in the list of germline variants;
   filtering out likely germline variants from the list of candidate somatic variants to retain only likely somatic variants,
   filtering out the likely germline variants further comprising the steps of:

      estimating, via a Hidden Markov Model (HMM) along each chromosome, a probability of each candidate somatic variant i being a germline variant ("Pgermline(i)"); and
      determining, based on the Pgermline(i), whether a candidate somatic variant i is germline or somatic, to retain only the likely somatic variants in the list of candidate somatic variants;

   determining the tumor mutational burden (TMB) value for the tumor sample based on a count of the retained likely somatic variants over all chromosomes.

2. The computer-implemented method of claim 1, wherein observed states for the HMM along each chromosome comprise variant fractions from the list of germline variants, and wherein the variant fractions from the list of germline variants are obtained from sequencing data.

3. The computer-implemented method of any preceding claim, wherein hidden states for the HMM along each chromosome comprise at least 9 discretized variant fractions.

4. The computer-implemented method of any preceding claim, wherein the HMM model employs a transition probability ($p_{switch}$) representing the probability that the two subsequent variants, $i$-1 and $i$, along a chromosome, with variant fractions, $VF_{i-1}$ and $VFi$, respectively, are associated to the same hidden state.

5. The computer-implemented method of claim 4, wherein $p_{switch}$ is chosen in a range from 1e-3 to 1e-6, preferably wherein $p_{switch}$ is a value of about 2e-4.

6. The computer-implemented method of any preceding claim, wherein emission probabilities of the HMM are defined as:

$$P\big(VF_O(i)\big|VF_H(i)\big) = 0.9 \times \text{Beta}\big((\alpha, \beta); VF_O(i)\big) + 0.1 \times \text{Uniform}\big(0, 1 ; VF_O(i)\big)$$

   wherein $\alpha = 1 + VF_H(i)$;
   wherein $\beta = 1 + D * (1 - VF_H(i))$;
   wherein $D$ is a mean coverage depth of variants in the tumor sample; and
   $VF_O(i)$ is an observed variant fraction originating from hidden state $VF_H(i)$.

7. The computer-implemented method of claim 6, wherein the mean coverage depth $D$ is rescaled by multiplying the mean coverage depth $D$ by a factor chosen in a range of 1/6 to 1/2.

8. The computer-implemented method of claim 7, wherein the mean coverage depth $D$ is rescaled by multiplying the mean coverage depth $D$ by a factor of 1/4.

9. The computer-implemented method of claim 6, 7 or 8, wherein the mean coverage depth $D$ is rescaled by multiplying the mean coverage depth D by a factor chosen in a range of 1/2 to 1.

10. The computer-implemented method of any preceding claim, wherein, for each chromosome, the HMM receives as input the list of heterozygous germline variants along said chromosome by retaining only those heterozygous germline variants observed with an observed variant fraction $VF_O(i)$ within a predetermined range of above 5% and below 90%.

11. The computer-implemented method of claim 9, wherein if the observed variant fraction $VF_O(i)$ of the heterozygous germline variants is equal to or greater than 50%, then the observed variant fraction $VF_O(i)$ is replaced by observed state value 1-VF.

12. The computer-implemented method of claim 1, further comprising adding one or more artificial data points at one or more chromosome genomic coordinates of regions with no observed variant calling data prior to estimating via the HMM.

13. The method of claim 11, wherein a regular grid is used in adding the one or more artificial data points, and wherein the regular grid comprises a resolution configured to maintain a predetermined number of grid points between a majority of observed variants.

14. The computer-implemented method of any preceding claim, wherein filtering out the likely germline variants from the list of candidate somatic variants comprises retaining only the candidate somatic variants for which the Pgermline(i) is below a threshold value (PgermlineThreshHMM), optionally wherein the PgermlineThreshHMM is 1e-3, preferably wherein the PgermlineThreshHMM is 1e-4.

15. The computer-implemented method of any preceding claim, further comprising reporting to an end user the TMB value for the tumor sample or wherein obtaining the variant calling data for the tumor sample comprises sequencing the tumor sample.

16. A computer-implemented method for selection for a cancer patient comprising the step of: determining a TMB value in the cancer patient tumor sample according to the computer-implemented method of claim 1.

**Patentansprüche**

1. Computerimplementiertes Verfahren zur Bestimmung eines Wertes der Tumormutationslast (TMB) in einer Tumorprobe, wobei das Verfahren umfasst:

   Erhalten von Variantenaufrufdaten für die Tumorprobe;
   Identifizieren einer Liste von Keimbahnvarianten für die Tumorprobe entlang jedes Chromosoms in den Variantenaufrufdaten und im Hinblick auf mindestens eine Populationsdatenbank,
   wobei jede der Keimbahnvarianten der Liste von Keimbahnvarianten eine Populations-Minor-Allel-Häufigkeit (pMAF) über einem Schwellenwert relativ zu der mindestens einen Populationsdatenbank aufweist;
   Identifizieren einer Liste von somatischen Kandidatenvarianten in den Variantenaufrufdaten,
   wobei die Liste von somatischen Kandidatenvarianten die verbleibenden Varianten in den Variantenaufrufdaten sind, die nicht in der Liste von Keimbahnvarianten identifiziert worden sind;
   Herausfiltern wahrscheinlicher Keimbahnvarianten aus der Liste von somatischen Kandidatenvarianten, um nur wahrscheinliche somatische Varianten zu behalten, wobei das Herausfiltern der wahrscheinlichen Keimbahnvarianten ferner die Schritte umfasst:

   Schätzen einer Wahrscheinlichkeit, dass jede somatische Kandidatenvariante i eine Keimbahnvariante ("Pgermline*(i)*") ist, über ein Hidden Markov Model (HMM) entlang jedes Chromosoms; und
   Bestimmen, basierend auf Pgermline(i), ob eine somatische Kandidatenvariante i eine Keimbahn- oder eine somatische Variante ist, um nur die wahrscheinlichen somatischen Varianten in der Liste von somatischen Kandidatenvarianten zu behalten;

   Bestimmen des Wertes der Tumormutationslast (TMB) für die Tumorprobe auf der Grundlage einer Zählung der zurückgehaltenen wahrscheinlichen somatischen Varianten über alle Chromosomen.

2. Computerimplementiertes Verfahren nach Anspruch 1, wobei die beobachteten Zustände für das HMM entlang jedes Chromosoms Variantenanteile aus der Liste von Keimbahnvarianten umfassen, und wobei die Variantenanteile aus der Liste von Keimbahnvarianten aus Sequenzierungsdaten erhalten werden.

3. Computerimplementiertes Verfahren nach einem der vorhergehenden Ansprüche, wobei die verborgenen Zustände für das HMM entlang jedes Chromosoms mindestens 9 diskretisierte Variantenanteile umfassen.

4. Computerimplementiertes Verfahren nach einem der vorhergehenden Ansprüche, wobei das HMM-Modell eine Übergangswahrscheinlichkeit ($p_{switch}$) verwendet, die die Wahrscheinlichkeit darstellt, dass die beiden aufeinander folgenden Varianten *i-1* und *i* entlang eines Chromosoms mit Variantenanteilen $VF_{i-1}$ bzw. $VF_i$ demselben verborgenen Zustand zugeordnet sind.

5. Computerimplementiertes Verfahren nach Anspruch 4, wobei $p_{switch}$ in einem Bereich von 1e-3 bis 1e-6 gewählt wird, wobei $p_{switch}$ vorzugsweise ein Wert von etwa 2e-4 ist.

6. Computerimplementiertes Verfahren nach einem der vorhergehenden Ansprüche, wobei die Emissionswahrscheinlichkeiten des HMM wie folgt definiert sind:

$$P(VF_O(i)|VF_H(i)) = 0{,}9 \text{ x } \text{Beta}((\alpha, \beta); VF_O(i)) + 0{,}1 \text{ x } \text{Uniform}(0, 1; VF_O(i))$$

wobei $\alpha = 1 + VF_H(i)$,
wobei $\beta = 1 + D * (1 - VF_H(i))$;
wobei D eine mittlere Abdeckungstiefe von Varianten in der Tumorprobe ist; und
$VF_O(i)$ ein beobachteter Variantenanteil ist, der aus dem verborgenen Zustand $VF_H(i)$ stammt.

7. Computerimplementiertes Verfahren nach Anspruch 6, wobei die mittlere Abdeckungstiefe *D* durch Multiplizieren der mittleren Abdeckungstiefe *D* mit einem Faktor, der in einem Bereich von 1/6 bis 1/2 gewählt wird, neu skaliert wird.

8. Computerimplementiertes Verfahren nach Anspruch 7, wobei die mittlere Abdeckungstiefe *D* durch Multiplizieren der mittleren Abdeckungstiefe *D* mit einem Faktor von 1/4 neu skaliert wird.

9. Computerimplementiertes Verfahren nach Anspruch 6, 7 oder 8, wobei die mittlere Abdeckungstiefe D durch Multiplizieren der mittleren Abdeckungstiefe D mit einem Faktor, der in einem Bereich von 1/2 bis 1 gewählt wird, neu skaliert wird.

10. Computerimplementiertes Verfahren nach einem der vorhergehenden Ansprüche, wobei das HMM für jedes Chromosom als Eingabe die Liste von heterozygoten Keimbahnvarianten entlang des Chromosoms erhält, indem es nur diejenigen heterozygoten Keimbahnvarianten beibehält, die mit einem beobachteten Variantenanteil $VF_O(i)$ innerhalb eines vorbestimmten Bereichs von über 5 % und unter 90 % beobachtet werden.

11. Computerimplementiertes Verfahren nach Anspruch 9, wobei, wenn der beobachtete Variantenanteil $VF_O(i)$ der heterozygoten Keimbahnvarianten gleich oder größer als 50% ist, der beobachtete Variantenanteil $VF_O(i)$ durch den beobachteten Zustandswert 1-VF ersetzt wird.

12. Computerimplementiertes Verfahren nach Anspruch 1, das ferner das Hinzufügen eines oder mehrerer künstlicher Datenpunkte an einer oder mehreren genomischen Chromosomenkoordinaten von Regionen ohne beobachtete Variantenaufrufdaten vor der Schätzung über das HMM umfasst.

13. Verfahren nach Anspruch 11, wobei beim Hinzufügen des einen oder der mehreren künstlichen Datenpunkte ein reguläres Gitter verwendet wird, und wobei das reguläre Gitter eine Auflösung aufweist, die so konfiguriert ist, dass eine vorbestimmte Anzahl von Gitterpunkten zwischen einer Mehrheit von beobachteten Varianten beibehalten wird.

14. Computerimplementiertes Verfahren nach einem der vorhergehenden Ansprüche, wobei das Herausfiltern der wahrscheinlichen Keimbahnvarianten aus der Liste von somatischen Kandidatenvarianten darin besteht, dass nur die somatischen Variantenkandidaten beibehalten werden, für die der Pgermline(i) unter einem Schwellenwert (PgermlineThreshHMM) liegt, wobei der PgermlineThreshHMM optional 1e-3 ist, vorzugsweise wobei der PgermlineThreshHMM 1e-4 ist.

**15.** Computerimplementiertes Verfahren nach einem der vorhergehenden Ansprüche, das ferner das Melden des TMB-Wertes für die Tumorprobe an einen Endbenutzer umfasst, oder wobei das Erhalten der Variantenaufrufdaten für die Tumorprobe das Sequenzieren der Tumorprobe umfasst.

**16.** Computerimplementiertes Verfahren zur Auswahl für einen Krebspatienten, das den Schritt umfasst: Bestimmen eines TMB-Wertes in der Tumorprobe des Krebspatienten nach dem computerimplementierten Verfahren nach Anspruch 1.

**Revendications**

**1.** Procédé mis en œuvre par ordinateur de détermination d'une valeur de fardeau mutationnel tumoral (TMB) dans un échantillon tumoral, le procédé comprenant :

l'obtention de données de détection de variants pour l'échantillon tumoral ;
l'identification, dans les données de détection de variants et compte tenu d'au moins une base de données de population, d'une liste de variants de lignée germinale pour l'échantillon tumoral le long de chaque chromosome, dans lequel chacun des variants de lignée germinale de la liste de variants de lignée germinale comporte une fréquence d'allèle mineur de population (pMAF) supérieure à un seuil par rapport à l'au moins une base de données de population ;
l'identification, dans les données de détection de variants, d'une liste de variants somatiques candidats, dans lequel la liste de variants somatiques candidats comporte les variants restants dans les données de détection de variants qui n'ont pas été identifiées dans la liste de variants de lignée germinale ;
le filtrage de variants de lignée germinale probables de la liste de variants somatiques candidats pour conserver uniquement des variants somatiques probables, le filtrage des variants de lignée germinale probables comprenant en outre les étapes de :

l'estimation, via un modèle de Markov caché (HMM) le long de chaque chromosome, d'une probabilité que chaque variant somatique candidat i soit un variant de lignée germinale (« Pgermline(*i*) ») ; et
la détermination, sur la base de Pgermline(*i*), si un variant somatique candidat *i* est de lignée germinale ou somatique, pour conserver uniquement les variants somatiques probables dans la liste de variants somatiques candidats ;

la détermination de la valeur de fardeau mutationnel tumoral (TMB) pour l'échantillon tumoral sur la base d'un comptage des variants somatiques probables conservés sur tous les chromosomes.

**2.** Procédé mis en œuvre par ordinateur selon la revendication 1, dans lequel les états observés pour le HMM le long de chaque chromosome comprennent des fractions de variant de la liste de variants de lignée germinale, et dans lequel les fractions de variant de la liste de variants de lignée germinale sont obtenues à partir de données de séquençage.

**3.** Procédé mis en œuvre par ordinateur selon une quelconque revendication précédente, dans lequel des états cachés pour le HMM le long de chaque chromosome comprennent au moins 9 fractions de variants discrétisées.

**4.** Procédé mis en œuvre par ordinateur selon une quelconque revendication précédente, dans lequel le modèle HMM emploie une probabilité de transition ($p_{switch}$) représentant la probabilité que les deux variants suivants, *i-1* et *i*, le long d'un chromosome, avec des fractions de variants, $VF_{i-1}$ et $VF_i$, respectivement, soient associés au même état caché.

**5.** Procédé mis en œuvre par ordinateur selon la revendication 4, dans lequel $p_{switch}$ est choisi dans une plage de 1e-3 à 1e-6, de préférence dans lequel $p_{switch}$ est une valeur d'environ 2e-4.

**6.** Procédé mis en œuvre par ordinateur selon une quelconque revendication précédente, dans lequel les probabilités d'émission du HMM sont définies comme suit :

$$P(VF_O(i)|VF_H(i)) = 0{,}9 \times \text{Bêta}((\alpha, \beta) ; VF_O(i)) + 0{,}1 \times \text{Uniforme}(0, 1 ; VF_O(i))$$

dans lequel $\alpha = 1 + VF_H(i)$ ;

dans lequel $\beta = 1 + D * (1 - VF_H(i))$ ;

dans lequel *D* est une profondeur de couverture moyenne de variants dans l'échantillon tumoral ; et

*VFo(i)* est une fraction de variant observée provenant de l'état caché *VF_H(i)*.

7. Procédé mis en œuvre par ordinateur selon la revendication 6, dans lequel la profondeur de couverture moyenne *D* est redimensionnée en multipliant la profondeur de couverture moyenne *D* par un facteur choisi dans une plage de 1/6 à 1/2.

8. Procédé mis en œuvre par ordinateur selon la revendication 7, dans lequel la profondeur de couverture moyenne *D* est redimensionnée en multipliant la profondeur de couverture moyenne *D* par un facteur de 1/4.

9. Procédé mis en œuvre par ordinateur selon la revendication 6, 7 ou 8, dans lequel la profondeur de couverture moyenne *D* est redimensionnée en multipliant la profondeur de couverture moyenne *D* par un facteur choisi dans une plage de 1/2 à 1.

10. Procédé mis en œuvre par ordinateur selon une quelconque revendication précédente, dans lequel, pour chaque chromosome, le HMM reçoit en entrée la liste des variants de lignée germinale hétérozygotes le long dudit chromosome en conservant uniquement les variants de lignée germinale hétérozygotes observés avec une fraction de variant observée *VFo(i)* dans une plage prédéterminée supérieure à 5 % et inférieure à 90 %.

11. Procédé mis en œuvre par ordinateur selon la revendication 9, dans lequel, si la fraction de variant observée *VFo(i)* des variants de lignée germinale hétérozygotes est supérieure ou égale à 50 %, alors la fraction de variant observée *VFo(i)* est remplacée par la valeur d'état observée 1-VF.

12. Procédé mis en œuvre par ordinateur selon la revendication 1, comprenant en outre l'ajout d'un ou de plusieurs points de données artificiels à une ou plusieurs coordonnées génomiques chromosomiques de régions sans données de détection de variants observées avant l'estimation via le HMM.

13. Procédé selon la revendication 11, dans lequel une grille régulière est utilisée pour ajouter les un ou plusieurs points de données artificiels, et dans lequel la grille régulière comprend une résolution configurée pour maintenir un nombre prédéterminé de points de grille entre une majorité de variants observés.

14. Procédé mis en œuvre par ordinateur selon une quelconque revendication précédente, dans lequel le filtrage des variants de lignée germinale probables de la liste des variants somatiques candidats comprend la conservation uniquement des variants somatiques candidats pour lesquels Pgermline(*i*) est inférieur à une valeur seuil (PgermlineThreshHMM), facultativement dans lequel PgermlineThreshHMM est 1e-3, de préférence dans lequel PgermlineThreshHMM est 1e-4.

15. Procédé mis en œuvre par ordinateur selon une quelconque revendication précédente, comprenant en outre le signalement, à un utilisateur final, de la valeur TMB pour l'échantillon tumoral ou dans lequel l'obtention des données de détection de variants pour l'échantillon tumoral comprend le séquençage de l'échantillon tumoral.

16. Procédé de sélection mis en œuvre par ordinateur pour un patient atteint d'un cancer comprenant l'étape de : la détermination d'une valeur TMB dans l'échantillon tumoral d'un patient atteint d'un cancer conformément au procédé mis en œuvre par ordinateur selon la revendication 1.

FIG.1

FIG.2

FIG.3

FIG.4a

FIG.4b

Before folding

a)

After folding

b)

FIG. 5

FIG. 6A

FIG. 6B

**FIG. 7**

**A** 20% Tumor purity (sequence20TP)

**B** 70% Tumor purity (sequence70TP)

FIG. 8

A — 20% Tumor purity (sequence20TP)

B — 70% Tumor purity (sequence70TP)

FIG. 9

**A** 20% Tumor purity (sequence20TP)

**B** 70% Tumor purity (sequence70TP)

**FIG. 10**

FIG. 11

700

702

703

704

705

Client
Devices:

710

Wireless
Network

Application Hosting
Server

713

706

712

Wide Area Network /
Local Area Network
(Network)

Client
Device

707

708

709

Server

Server

Server

**FIG. 12**

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018106884 A
- WO 2020092591 A
- WO 2017151524 A
- US 2019362808 A1
- WO 2017220508 A

**Non-patent literature cited in the description**

- **SNYDER et al.** *N England J Med*, 04 December 2014, vol. 371 (23)
- **RIVZI et al.** *Science*, 03 April 2015, vol. 348 (6230)
- **CAMPESATO et al.** *Oncotarget*, 2015, vol. 6 (33)
- Aligning Tumor Mutational Burden (TMB) quantification across diagnostic samples: Phase 2 of the Friends of Cancer Research TMB harmonization project. *Annals of Oncology*, 30 September 2021
- **KESSLER et al.** *Nature Comms*, 11 October 2016, vol. 12521 (7)
- **GAROFALO et al.** The impact of tumor profiling approaches and genomic data strategies for cancer precision medicine. *Genome Medicine*, 2016, vol. 8, 79
- **SUN et al.** *PLoS Comput Biol*, 2018, vol. 14 (2)
- **COLLINS**. Encyclopedia of Biometrics. 2009